# EUROPEAN PATENT SPECIFICATION

(11) **EP 4 478 968 B1**
(45) Date of publication and mention of the grant of the patent: **30.09.2026**
(21) Application number: 23705392.1
(22) Date of filing: 15.02.2023
(51) Int. Cl.: A61B 17/70

(54) **SPINAL BONE FASTENER ASSEMBLY**
WIRBELSÄULENKNOCHENBEFESTIGUNGSANORDNUNG
ENSEMBLE DE FIXATION D'OS VERTÉBRAL

(30) Priority: 18.02.2022 CH 1672022
(43) Date of publication of application: 25.12.2024
(73) Proprietor: AO Technology AG, 7000 Chur (CH)
(72) Inventor: KOLLER, Heiko, 6322 Kirchbichl (AT); OVERES, Tom, 4536 Attiswil (CH)
(74) Representative: Ricker, Mathias
(86) International application number: PCT/EP2023/053715
(87) International publication number: WO 2023/156431

(56) References cited:
- US-A1- 2008 221 621
- US-A1- 2015 164 561
- US-B2- 10 292 737
- US-B2- 9 855 077

## Description

### TECHNICAL FIELD

The present invention relates to a spinal bone fastener assembly.

### PRIOR ART

In orthopaedic surgery around the spine, posterior spinal stabilization systems are often placed to a target site to realign, correct and/or stabilize the spinal column to compensate for malalignment caused by for example degeneration of the spine, born malalignments, such as excessive lordosis, kyphosis and scoliosis, and for example trauma, such as fractures.

A typical posterior spinal stabilization system consists of multiple pedicle screws that are connected by a rod. In a first step, at different levels of the spinal column, the pedicle screws are screwed through the pedicle bone into vertebral bodies. In a second step, the pedicle screws are connected by a rod, having a desired shape or curvature. Using a spinal column persuading instrument, the vertebral bodies including the pedicle screws are forced towards the rod (or vice versa). When the rod has reached the end position within a rod receiving head of the pedicle screw, the rod is rigidly fixated by tightening a setscrew against the rod. Due to this immobilization, the involved levels of the spinal column will start to fuse.

The spinal column comprises multiple vertebral levels which are separated by an intervertebral disc. The intervertebral disc provides a cushioning effect and due to its flexibility will allow a person to bend the spine. When multiple vertebral bodies are connected by a posterior spinal system, the involved levels will not be able to execute this function. As a result, the adjacent or neighboring levels will need to partly compensate this function.

As a result, at the sudden transition of the mobile spinal levels to a rigid and long multilevel screw-rod construct higher stresses will occur and can result into complications such as a new pathological kyphosis starting at the neighboring level.

As a result, for example proximal junction kyphosis (PJK) and distal junction kyphosis (DJK) and screw pull out are the major complications and challenges with adult multilevel spinal fusion surgeries. Often the complications lead to the need of revision surgery.

To reduce the risk of PJK and DJK, the use of more dynamic transition implants, such as tethers (a non-fusion spinal device intended to treat idiopathic scoliosis) or hooks, instead of rigid pedicle screws, has shown to decrease the risk for implant related failures, such as pull-out. But both of these approaches have technique-related disadvantages. In particular, tethers are required to be passed under the lamina or around the spinal processes, tensioned and then tied off. This requires visualization of the site and involves harm to the surrounding soft tissues and bears the risk of injury to the dura or spinal cord when tethers are passed under the lamina. Moreover, these steps increase the complexity and duration of the surgery.

Another common cause of complications is the fixation of the rod to the most proximal screw. This fixation step usually requires great forces as the rod is rarely perfectly bent and this can create stresses at the screw-bone interface. These stresses can initiate screw-bone loosening. Also, in osteoporotic bone surgeries and in spinal deformity surgeries, perfect rod bending to match the position is often challenging, given the limited correction, in particular in rigid spinal deformities. Osteoporotic bones and high stresses impacting the screw-bone interface can cause screw loosening, particularly at the apex of deformities and at any level where there is a greater offset between the screw head locking position and the rod.

A spinal bone fastener assembly to be engaged to a posterior spinal bone fastener system with the features of the preamble of claim 1 is known from US 9,855,077 B2.

A further spinal implant comprising a fastener including a head and defining a longitudinal axis is known from US 10 292 737 B2. This document describes a member configured for disposal of the head and defining an inner groove configured for disposal of a band that is engageable with the head to connect the fastener and the member. The member is moveable relative to the longitudinal axis. The member defines a passageway configured for disposal of a tether.

### SUMMARY OF THE INVENTION

Thus, in view of the above, there is a need for an improved spinal bone fastener assembly that decreases the stresses on the pedicle screw-bone interface, especially at the transition from the mobile spine to the rigid screw-rod construct, and so provides a smoother transition from the instrumented levels to the adjacent levels above and / or below. Such a spinal bone fastener assembly should be compatible with the screw-rod construct used to treat the spinal pathology, should be possible to apply causing minimal harm to the soft tissues, should allow variable adjustment of the stiffness and distance between the spinal column and the connecting rods to accommodate for individual differences in a patient's anatomy, and should not require manipulations, which involve tying off or tensioning tethers by hand, leading to irreproducible outcomes.

The present invention is defined in claim 1 and claim 23 with preferred embodiments detailed in the dependent claims. To facilitate understanding of the invention, associated methods are also described herein. However, these methods do not form part of the claimed invention.

The invention provides inter alia a spinal bone fastener assembly to be engaged to a posterior spinal bone fastener system, the spinal bone fastener assembly comprising: a bone fastener having a bone fastener head portion, a bone engaging elongated shaft and a first drive for introducing the bone fastener into a target bone, a rod receiving head configured to receive and at least partially encompass a spinal rod in a first rod receiving opening with a central pocket having a clamping seat, a tightening means configured to exert a retaining and/or clamping force between the spinal rod and the rod receiving head and at least one flexible elongate member connecting or coupling the rod receiving head with the bone fastener. The tightening means preferably comprises a main body with a tightening means top-side and a tightening means bottom side. The bone fastener further comprises at least one first flexible elongate member retaining means. The rod receiving head preferably comprises a head top end and a head bottom end. The first rod receiving opening in the rod receiving head preferably extends from a front side to a backside. The rod receiving head moreover comprises at least one second flexible elongate member retaining means and a first rod setting or tightening mechanism. The tightening means comprises a second drive to engage the tightening means into the rod receiving head. The spinal bone fastener assembly has a second tightening mechanism sized and shaped to engage with the first rod setting or tightening mechanism. This second tightening mechanism can be realized within the at least one tightening means. The flexible elongated member comprises a long elongated flexible body which is sized and shaped to engage with first and second flexible elongate member retaining means and of which at least a portion spans between the bone fastener head portion and the rod receiving head bottom end, wherein the bone fastener head portion is spaced at a distance from the rod receiving head bottom end. The engagement comprises a clamping of a section of the elongated flexible body within the rod receiving head.

The spinal bone fastener assembly has a locked configuration and an unlocked configuration, wherein in the locked configuration the elongated flexible member is biased against the clamping seat and translation of the flexible elongate member through at least one of the first and second flexible elongate member retaining means is inhibited. In the unlocked configuration the elongated flexible member can be displaced and especially the portion spanning between the bone fastener head portion and the rod receiving head bottom end can be adjusted, wherein the bone fastener head portion can be spaced at different predetermined distances from the rod receiving head bottom end.

In other words, a spinal bone fastener assembly to be engaged to a posterior spinal bone fastener system comprises a bone fastener, a rod receiving head, configured to receive a spinal rod, a tightening means configured to exert a retaining and/or clamping force between the spinal rod and the rod receiving head, a tether connecting or coupling the rod receiving head with the bone fastener. When the bone fastener is introduced into the spine, a spinal rod is positioned in a central pocket of the rod receiving head and a rod tightening means tightens against the spinal rod in the central pocket blocking the position of the tether in the tether retaining means of the rod receiving head at a predetermined distance.

The first and second tightening mechanisms can be configured as internal and/or external threads.

At least one of the first or second flexible elongate member retaining means can be oversized in comparison to the cross-section of the flexible elongate member.

At least one of the first or second flexible elongate member retaining means preferably fully encompasses the flexible elongate member to inhibit separation thereof. The flexible elongate member retaining means can comprise one or more passages in the bone fastener head portion as well as in rod receiving head bottom end. Preferably, two parallel passages are provided in the bone fastener head portion, In the rod receiving head bottom end there are preferably two essentially parallel sequences of passages from the outside of the rod receiving head into its central pocket from one side and from the central pocket towards the outside of the rod receiving head, wherein in the central pocket said tightening means are configured to engage two parallel oriented corresponding portions of the flexible elongate member.

At least one of the first flexible elongate member retaining means can be directed top to bottom, which can also be defined as distally to proximally, or are directed side to side.

A tightening means can be configured as a main tightening means sized and shaped to receive an inner tightening means. The inner and main rod tightening means can then be threadedly engaged by means of an inner thread and an outer thread.

**In** said locked configuration the main tightening means can be abutting with the spinal rod and the flexible elongate member or tether is abutting with the spinal rod and the clamping seat.

The rod receiving head can comprise an end seat which forms a depth insertion depth stop for the main rod tightening means which allows that the spinal rod is not blocked in its longitudinal movement, when the main rod tightening means is fully tightened.

An intermediate inlay can be arranged between the clamping seat and at least one of the main or inner tightening means to separate the action of the main and inner tightening means allowing to reach the blocking configuration of the flexible elongate member independently from the adjustment of the position of the spinal rod. Especially, in said locked configuration the inner tightening means can be abutting with the spinal rod and the flexible elongate member or tether is abutting with the intermediate inlay and the clamping seat. In another configuration of the intermediate inlay, in said locked configuration, the inner tightening means can be abutting with the intermediate inlay and the flexible elongate member is abutting with the intermediate inlay and the clamping seat.

It is also possible that in said locked configuration the spinal rod is abutting with the first rod receiving opening end and the main tightening means bottom side.

The outer periphery of the bone fastener head portion can be predominantly non-circular or non-rotational symmetric, e.g. a cuboid. Then the first flexible elongate member retaining means can be one central or two parallel passages or throughbores perpendicular to the longitudinal direction of the bone fastener or the first flexible elongate member retaining means can be e.g. four parallel passages or throughbores in parallel to the longitudinal direction of the bone fastener in the corners of the cuboid.

The first drive can comprise an internal thread for attachment of a bone cement insertion cannula.

Relating to the material of the flexible elongated member, it can be made of woven or braided strands of a biocompatible surgical suture material such as UHMWPE fibres, nylon fibres, Polyester fibres, PET fibres, or Stainless Steel, Cobalt Chromium or titanium(alloy) cables, as well as polyaramide, carbon fibres or silk.

The flexible elongate member can be a round cable or a band. It can be mounted in a symmetrical way or on one side of the rod receiving head.

The bone fastener can comprise an insertion depth stop arranged adjacent to the distal side of the bone fastener head portion and wherein said insertion depth stop is configured to inhibit a too deep insertion into the bone.

The rod receiving head can comprise a rod tightening means receiving extensions that project from a head top end of the rod receiving head. Such two extensions on the head top ends preferably each have a breaking groove at the corresponding head top end so that the extensions can be removed when the rod tightening means are positioned on the spinal rod.

The rod receiving head can comprise a first drive shaped aperture that extends through the head bottom end allowing to introduce a screwdriver through the rod receiving head for fixing the bone fastener in the bone while the rod receiving head is already connected via the connecting flexible elongate members with the bone fastener head.

The flexible elongated member preferably has a distal coupling end for attachment to an insertion device, especially allowing to adjust the span distance between the bottom of the rod receiving head and the head of the bone fastener in the unlocked configuration.

When an intermediate inlay is provided, it can comprise a second rod receiving opening with a first depth 'D1' which is smaller than the spinal rod diameter, or with a second depth 'D2' is which larger than the spinal rod diameter, allowing either to block any longitudinal movement of the spinal rod or to the contrary allowing a longitudinal movement of the spinal rod.

The flexible elongated member can be entering or exiting the rod receiving head from the head bottom end and/or entering or exiting the rod receiving head from the head outer wall.

The invention further comprises a kit comprising a spinal bone fastener assembly as mentioned before as well as an insertion device including a tensioning mechanism to tension the flexible elongate member. The tensioning mechanism comprises a tether holding element as e.g. a hook which travels alongside the insertion device in the longitudinal direction of the bone fastener adjusting the distance between the bone fastener head and the rod receiving head.

Such a kit can comprise a tensioning force measuring device so that the adjusting the distance can be controlled to a specific tension force.

The above mentioned spinal bone fastener assembly can be used within a surgical method for engaging a spinal bone fastener assembly to a posterior spinal bone fastener system, wherein the method comprises: providing such a spinal bone fastener assembly, inserting a bone fastener into the spine; while the rod receiving head is positioned in a predetermined distance from the bone fastener head portion, especially in contact for the respective complementary surfaces, introducing a spinal rod in a central pocket of the rod receiving head and tightening a rod tightening means against the spinal rod in the central pocket blocking the position of the tether in the tether retaining means of the rod receiving head. In a preferred variant of the inner tightening means is only tightening the intermediate inlay to the tether and not touching the rod.

When the spinal bone fastener assembly comprises a spindle tensioning mechanism configured to move a tether holding hook in the longitudinal direction of the bone fastener, wherein during the tightening step of the tightening means against the spinal rod in the central pocket the rod receiving head bottom end is moved relatively to the spinal rod and the tightening step is stopped before blocking the position of the tether in the tether retaining means of the rod receiving head, and comprising the further step of actuating the spindle tensioning mechanism to tension the or tether positioned around the tether holding hook and position the bone fastener head portion at a predetermined distance from the rod receiving head bottom end before finishing the tightening step by means of an inner and/or main tightening means to block the position of the flexible elongate member in the tether retaining means of the rod receiving head.

The spinal bone fastener assembly may be used in a spinal implant, which is dedicated to reducing the stress after correcting the spinal column and to preventing proximal and distal junctional kyphosis..

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1A: shows a perspective view of a spinal bone fastener assembly in a preassembled state without a spinal rod;
- Fig. 1B: shows an exploded view of the elements of Fig. 1A;
- Fig. 2A: shows a perspective view of the bone fastener of Fig. 1A;
- Fig. 2B: shows a perspective view on the head of the bone fastener of Fig. 2A;
- Fig. 2C: shows a side view of the bone fastener of Fig. 2A;
- Fig. 2D: shows a cross-section view of the bone fastener of Fig. 2C along line 2C-2C;
- Fig. 3A: shows a perspective view of the rod receiving head of Fig. 1A;
- Fig. 3B: shows a different perspective view of the rod receiving head of Fig. 3A;
- Fig. 3C: shows a perspective view of the rod receiving head of Fig. 1A;
- Fig. 4A: shows a perspective view of a main tightening means;
- Fig. 4B: shows a further perspective view of a main tightening means;
- Fig. 5A: shows a perspective view of an inner tightening means;
- Fig. 5B: shows a further perspective view of an inner tightening means;
- Fig. 6A: shows an exploded view of the tightening means of Fig. 4A and 5A;
- Fig. 6B: shows a partial cross-section view of the tightening means of Fig. 4A and 5A with the inner tightening means not yet fully screwed in;
- Fig. 6C: shows a partial cross-section view of the main tightening means of Fig. 4A and 5A with the inner tightening means screwed beyond the lower surface;
- Fig. 7A: shows a perspective view of the disposition of the flexible elongated member or tether without showing the other elements of the spinal bone fastener assembly;
- Fig. 7B: shows a different perspective view of the tether;
- Fig. 8A to 8F: show sequences of the different steps of mounting of a rod into the spinal bone fastener assembly;
- Fig. 8G: shows a perspective view of a spinal bone fastener assembly similar to Fig. 8A with an internal second tightening means;
- Fig. 8H: shows a perspective view of the spinal bone fastener assembly with removed sidewalls;
- Fig. 8I: shows a perspective view of a spinal bone fastener assembly as in Fig. 8A with an internal second tightening means allowing a longitudinal movement of the spinal rod;
- Fig. 8J: shows the perspective view of Fig. 8I with an inner tightening means blocking the spinal rod;
- Fig. 9A: shows an exploded view of a variant spinal bone fastener assembly similar to the assembly according to Fig. 1A with an additional intermediate inlay for a growth guiding application;
- Fig. 9B: shows a perspective view of Fig. 9A with the intermediate inlay placed in the rod receiving head;
- Fig. 10A to 10D: show perspective views of the intermediate inlay of Fig. 9A as well two side views of two different heights of the intermediate inlay for the standard tether spinal bone fastener application and the growth guiding application;
- Fig. 11A to 11H: show a plurality of perspective views of the introduction and fixation of a spinal rod in the variant spinal bone fastener assembly with the intermediate inlay;
- Fig. 12A to 12D: show a plurality of perspective views of the introduction and fixation of a spinal rod in the variant spinal bone fastener assembly with two different intermediate inlays;
- Fig. 13A to 13D: show an assembly of the insertion device to the spinal bone fastener assembly;
- Fig. 14A to 14Y: show the implantation of the spinal bone fastener assembly in a spine of a patient;
- Fig. 15A and 15B: show perspective views of variant bone fastener;
- Fig. 16A: shows a perspective view a front-loading rod receiving head;
- Fig. 16B: shows a perspective view a side-loading rod receiving head;
- Fig, 17A and Fig. 17B: show a variant with double rod receiving head to receive two spinal rods;
- Fig. 18A to 18G: show a variant of the tether disposition in the rod receiving head and a variant of the clamping mechanism;
- Fig. 19A to 19C: show a tether clamping step with a variant tightening means;
- Fig. 20A to 20D: show a growth guiding variant of the variant of Fig. 18 and 19;
- Fig. 21A to 21C: show a double tether band and intermediate inlay providing for a double sided tensioning;
- Fig. 22A to 22C: show a double tether band at the fastener and intermediate inlay providing for a single sided tensioning;
- Fig. 23A to 24C: show a single tether band for the fastener and intermediate inlay providing for a single sided tensioning;
- Fig. 24A to 24C: show a single tether band for the fastener providing for a single sided tensioning without an intermediate inlay;
- Fig. 25A to 25C: show two single tether band for the fastener providing for a double sided tensioning with an intermediate inlay;
- Fig. 26A and 26B: show a double sided insertion device having two tether holding hooks; and
- Fig. 27: shows a variant of the spinal bone fastener assembly comprising a spacer between the bone fastener and the rod receiving head.

### DESCRIPTION OF PREFERRED EMBODIMENTS

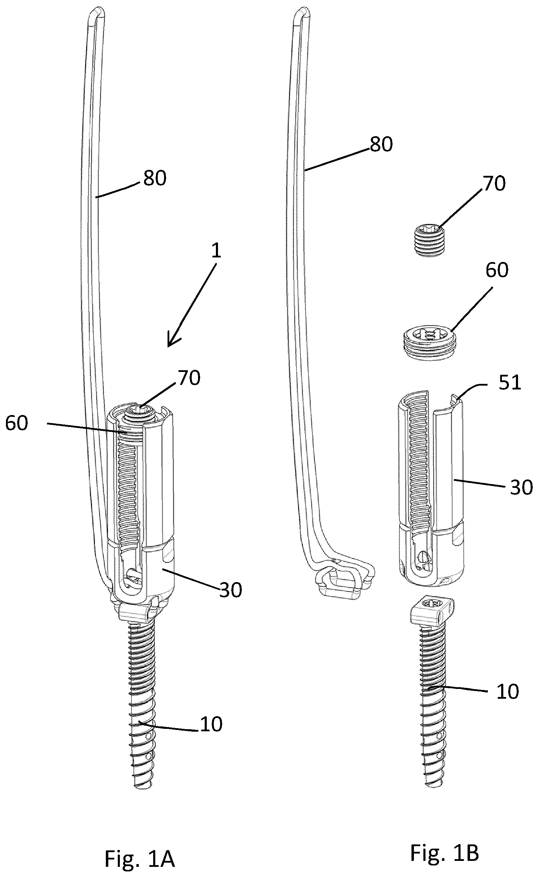

Fig. 1A shows a perspective view of a spinal bone fastener assembly 1 in a preassembled state without a spinal rod 2. Fig. 1B shows an exploded view of the elements of Fig. 1A.

The spinal bone fastener assembly comprises
- a bone fastener 10, more specifically a spinal pedicle screw for engagement into a target vertebral body,
- a rod receiving head 30, configured to receive and at least partially encompass a spinal rod 2,
- at least one flexible elongate member 80, called tether in the present description, connecting or coupling the rod receiving head 30 with the bone fastener 10 in a flexible manner,
- at least one main tightening means 60 for setting or fixation of the rod and/or tether, and
- at least one inner tightening means 70 for fixation of the rod and/or tether.

As described in greater detail later, the spinal bone fastener assembly is intended to be combined with a longer posterior spinal stabilization construct consisting of state-of-the art pedicle screws and posterior rods.

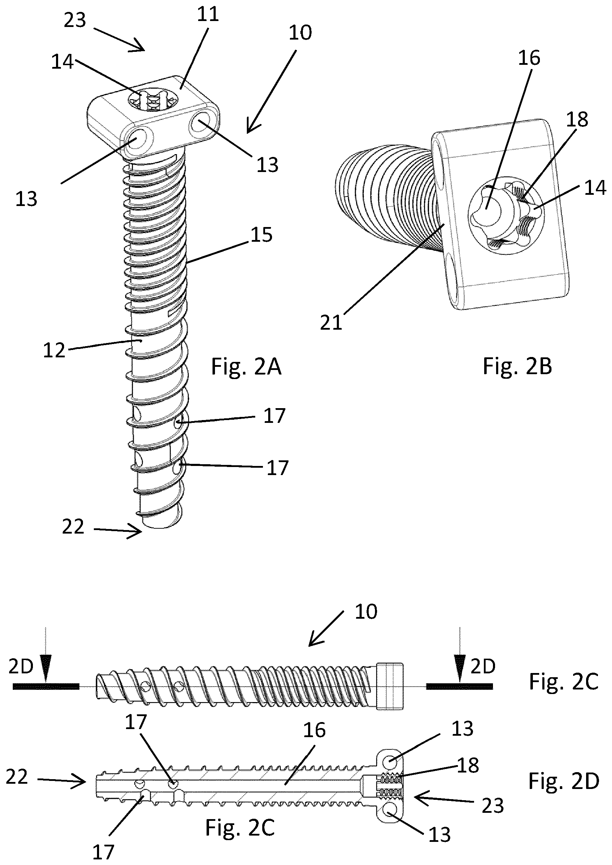

Referring to Figs. 2A-2D, the bone fastener 10 is shown in greater detail. Fig. 2A shows a perspective view of the bone fastener 10 of Fig. 1A; while Fig. 2B shows a perspective view on the head of the bone fastener 10 of Fig. 2A; while Fig. 2C shows a side view of the bone fastener 10 of Fig. 2A providing a cutting plane line 2D-2D used in Fig. 2D for a cross-section view of the bone fastener of Fig. 2C along said line 2D-2D;

Bone fastener 10 comprises a bone fastener head portion 11 and a bone engaging elongated shaft 12. The end of the shaft forms the distal end 22 or tip of the bone fastener, and the top end of the head portion 11 forms the proximal end 23 of the bone fastener. The elongated shaft 12 comprises an external thread 15 for fixation in the target bone. The bone fastener head portion 11 comprises an internal first drive 14 for engagement with an insertion aid such as a screwdriver.

The bone fastener head portion 11 furthermore comprises at least one first flexible elongate member retaining means 13. In this example, the first flexible elongate member retaining means 13 are configured as two horizontal bores or channels extending through the head portion 11 and are arranged aside and adjacent to the first drive 14. The term horizontal means substantially perpendicular to the longitudinal axis of the screw. The first flexible elongate member retaining means 13 are configured to at least encompass portions of a tether 80. For wear reduction and friction reduction purposes, the edges of the first flexible elongate member retaining means 13 are blended or chamfered. As depicted, the head portion 11 has a larger periphery than the elongated shaft 12 and hence forms a mechanical depth stop 24 to inhibit of too deep insertion of the bone fastener into the target bone. On the contrary to standard pedicle screws, the bone fastener head portion 11 is predominantly non-circular or non-rotational symmetric and provides sufficient space for the first flexible elongate member retaining means 13.

The spinal bone fastener 10 is intended to be connected to the rod receiving head 30 and by a flexible elongate member 80. Due to the flexible connection predominantly tensile forces will be transferred to the spinal bone fastener 10 and bone interface. For this purpose the external threads may have an asymmetric crest shape configured to specifically withstand tensile forces by for example having a flat proximal angle and a more steep distal angle.

In this example the drive is shaped as hexa-lobe or 'torx' drive. The first drive 14 as shown in Fig. 2B is shaped as a bore with circumferentially arranged recesses.

Typically, when implanting a bone fastener in osteoporotic bone, bone cement is injected through a bone fastener to cure around the distal end 22 of the bone fastener and so to increase the primary stability in the osteoporotic bone. For this purpose, the bone fastener 10 comprises a central cannulation 16 with intersecting fenestrations 17 along the distal section 25 of the elongated shaft 12. To inhibit bone cement leakage, the first drive 14 may comprise an internal thread 18 for rigid attachment of a bone-cement injecting device.

Often, a central cannulation 16 moreover forms a guiding channel that allows the bone fastener 10 to be inserted into the target bone over a previously placed guide-wire or Kirschner wire. The upper surface of the head 11 can be a flat surface.

The bone fastener 10 can comprise one central single horizontal channel in the centre crossing the recess of the first drive 14. The tether retaining means are not necessary a round bore, it can be a slotted hole or other formed channel to receive e.g. a band as tether means.

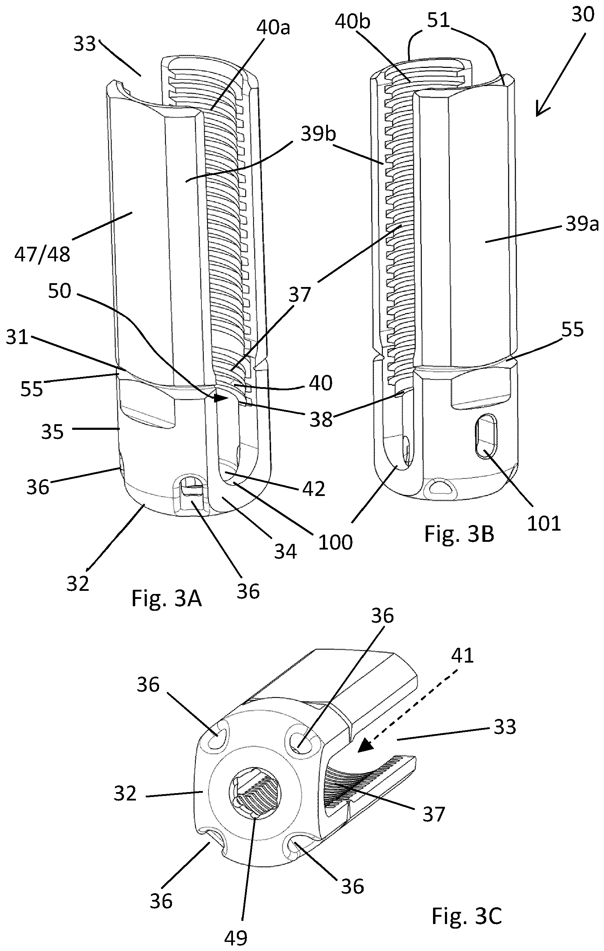

Fig. 3A, 3B and 3C show three different perspective views of the rod receiving head 30 of Fig. 1A. The rod receiving head 30 comprises a head top end 31 and a head bottom end 32. The rod receiving head 30 has a first rod receiving opening 33 starting at the top end 31 and extending from a front side 34 to a backside 35. The first rod receiving opening extends towards the head bottom end and ends adjacent to the bottom end and so defining a first rod receiving opening end 100. As shown, the rod receiving opening end is semi-circular shaped, but alternatively may be flat, flat with corner radii, V-shaped etc.. A preferred semi-circular shape provides a most-material condition and so increases the stability of the rod receiving head. Said head bottom end 32 is arranged to face the proximal end 23 of the bone fastener 10. In this example the bone fastener proximal end 23 and the head bottom end 32 are shaped as flat surfaces.

In other words, the rod receiving head 30 has a U-shape which extends away from the head bottom end 32.

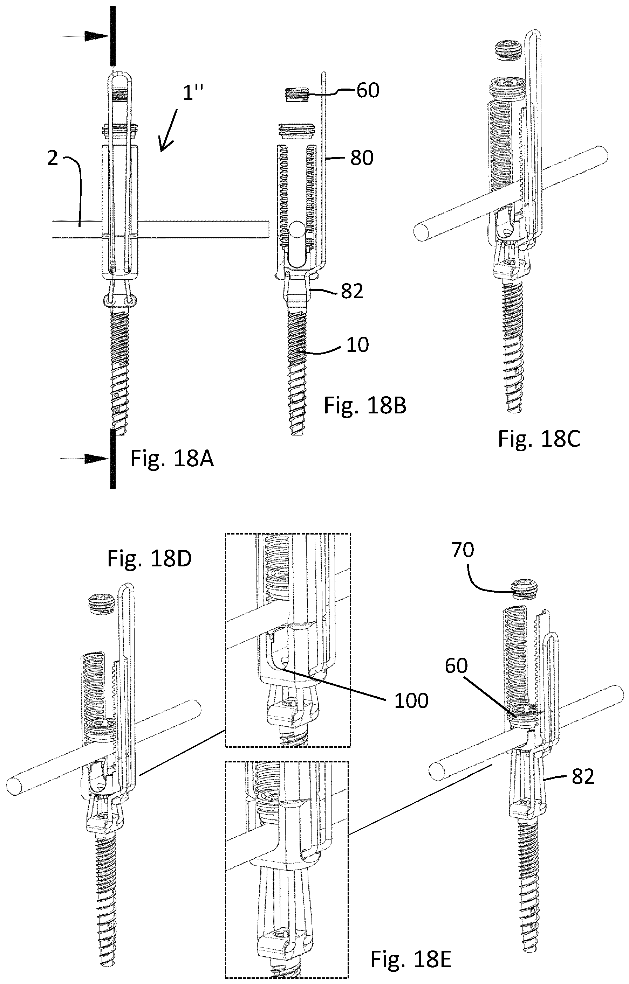

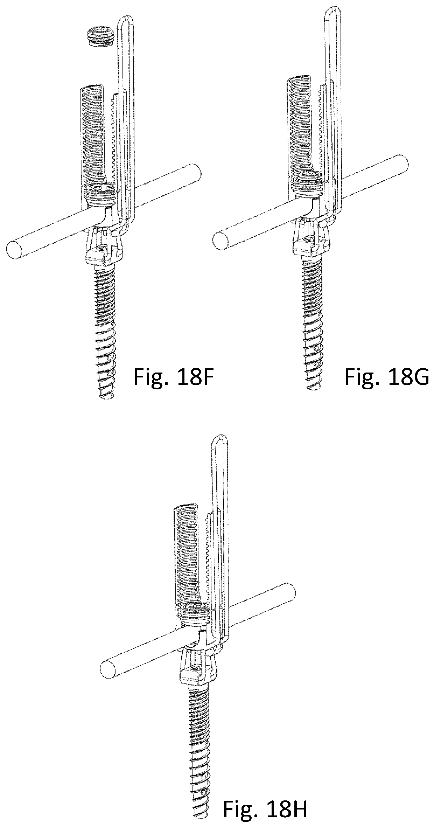

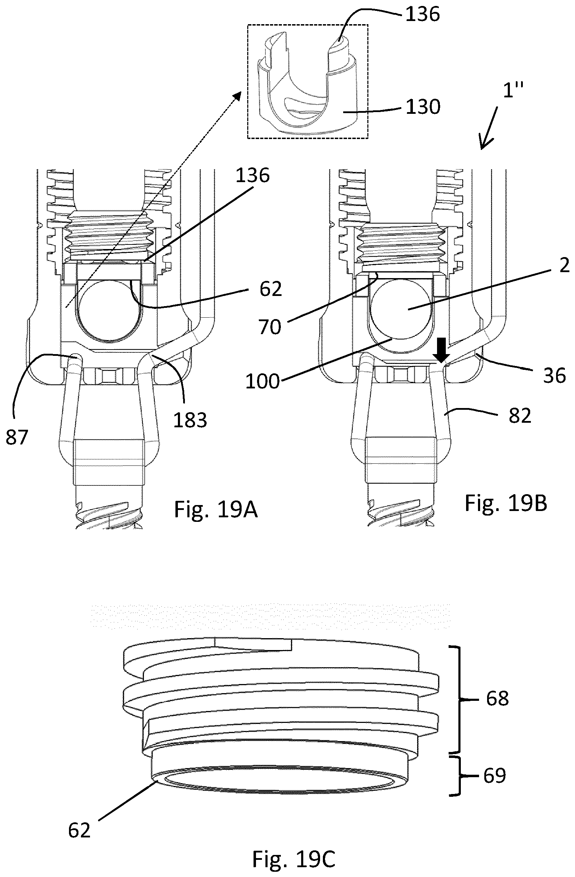

The rod receiving head moreover comprises a blind central pocket 41 which intersects the first rod receiving opening 33 and also extends from the head top end 31 towards the head bottom end 32. Adjacent to the head bottom end 32, the central pocket 41 has a lower surface defining a clamping seat 42. Alternatively the clamping seat may be formed by the sidewalls 43 of the central pocket 41 as described later for Figs. 18 and 19. At least a portion of the central pocket side walls 43 are configured as a first rod setting or tightening mechanism portion 37, for example as an internal thread 40a, 40b which is sized and shaped to receive the main tightening means 60. The central pocket 41 may be sized and shaped as a stepped central pocket 41, comprising a stepped section 50 that defines an end seat 38. This end seat 38 may limit the insertion depth of a main tightening means 60 as described in greater detail later. The rod receiving head 30 furthermore comprises at least one second flexible elongate member retaining means 36 configured as a passage extending from the lower outside or head bottom end 31 into the clamping seat area and intersecting therewith. The second flexible elongate member retaining means 13 are configured to at least partly encompass portions of a tether 80.

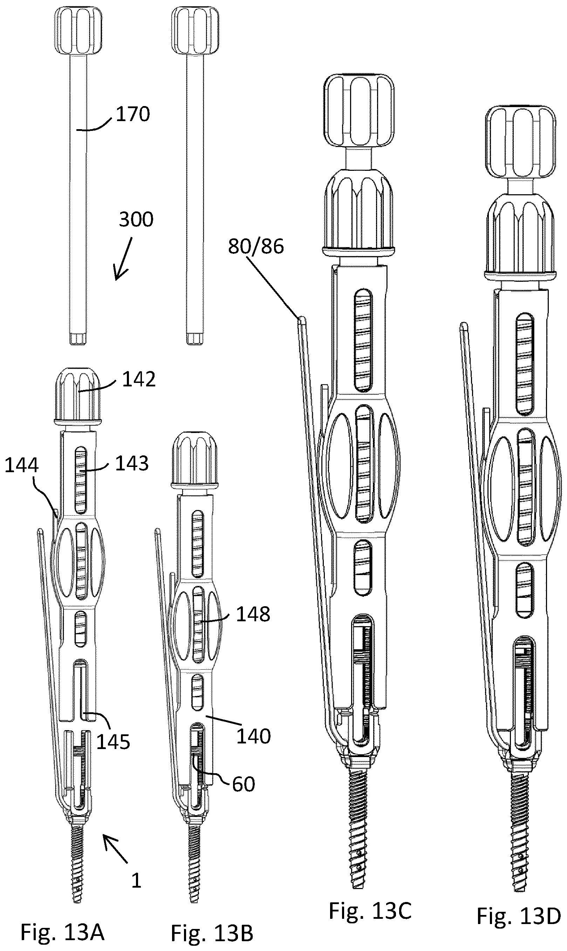

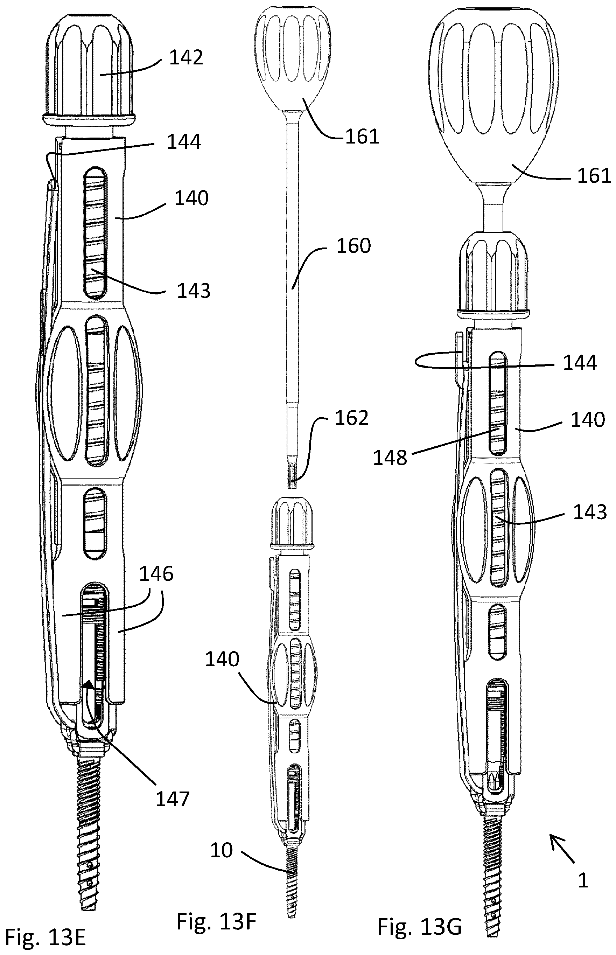

Two extensions 39a and 39b are provided at the free ends 31 of the generally U shaped rod receiving head and projecting thereof in a proximal direction. At the area of connection or start of projection, the rod receiving head 30 and/or extensions 39 comprises internal and/or external breaking grooves 55. In one embodiment the first rod setting or tightening mechanism portion 37 extends into the extensions towards the extension end 51. The extensions facilitate ease of insertion of the posterior rod during surgery and are removed by breaking or cutting, as described for the surgical steps of Figs. 13.

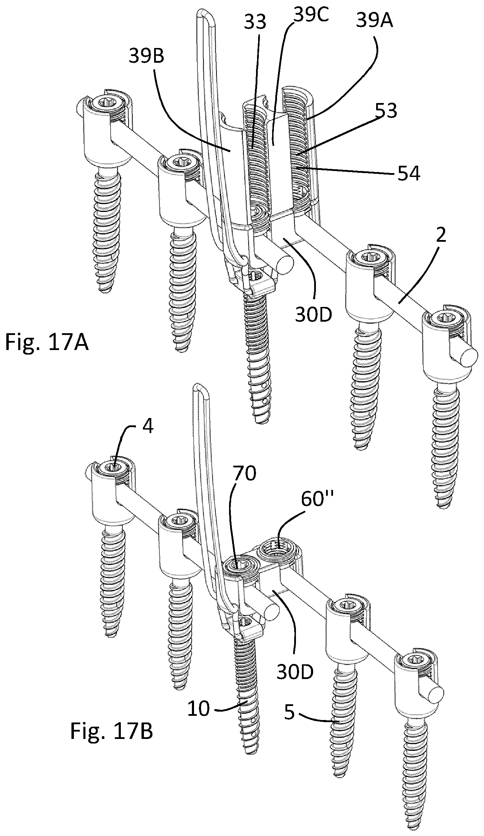

The central pocket 41 can also have a clamping seat 42 at the bottom of the U shaped head. The extensions can also have a double sided middle extension 39c as shown in the embodiment of Fig. 17A.

The rod receiving head 30 and/or main tightening means receiving extensions 39a, 39b comprises an anti-rotation element 47 such as a flat face 48 which is sized and shaped to mate with an internal wall 145 of an insertion device 140 and so to provide rotational stable coupling between the spinal bone fastener assembly 1 and the insertion device 140. As described for Figs. 13A to 13G, the rotationally stable coupled insertion device allows the surgeon to counter or oppose the torque applied to the full spinal construct during the final tightening step of the inner tightening means 70 and/or main tightening means 60.

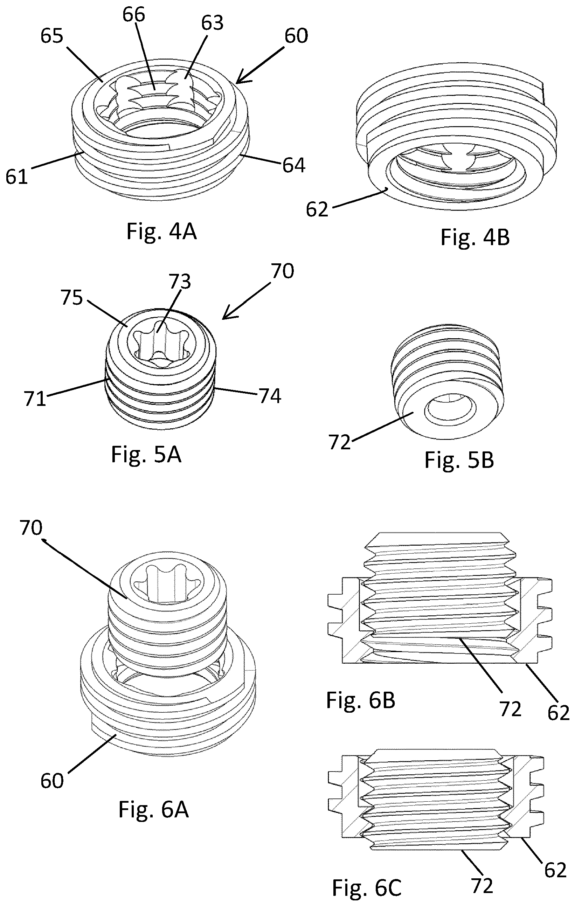

Fig. 4A and 4B show perspective views of a main tightening means 60. Fig. 5A and 5B show perspective views of an inner tightening means 70. Fig. 6A shows an exploded view of the tightening means 60 and 70 of Fig. 4A and 5A. Fig. 6B and 6C show a partial cross-section view of the tightening means 60 and 70 of Fig. 4A and 5A with the inner tightening means 70 screwed in the main rod tightening means 60 at different depths.

The main tightening means 60 comprises a first main body 61 having a main tightening means top-side 65 and a main tightening means bottom side 62. Inner recesses form a second drive 63, for example a hexa-lobe drive, to engage the main tightening means 60 into the rod receiving head 30. The main tightening means 60 comprises a main second tightening mechanism portion 64 which is sized and shaped to engage with the first rod setting or tightening mechanism portion 37. The second rod tightening mechanism portion 64 is shaped as an external thread, and hence the main rod tightening means 60 is configured as a first grub screw. The combined first and second tightening mechanism portions 37, 64 form a tightening mechanism or a coupling between the main tightening means and the rod receiving head. As described, in this example the tightening mechanism is a threaded. A coupling may also be achieved by for example a bayonet coupling, a friction coupling, a pressfit-coupling, a snap-fit coupling, a morse-taper coupling, and etc..

In this example, the main tightening means 60 furthermore comprises a throughbore 67 which at least partially comprises an inner thread 66, sized and shaped to receive the inner tightening means 70.

The inner tightening means 70 comprises a second main body 71 with an inner tightening means top-side 75 and an inner tightening means bottom side 72. Inner recesses form a third drive 73 configured to be engaged with an insertion instrument such as a screwdriver. In this example the third drive is configured as a hexa-lobe drive. More specifically the third drive is similarly sized and shaped as the first drive 14, and therefore can be operated with the same screwdriver. The inner tightening means furthermore comprises an outer second tightening mechanism 74 which is sized and shaped to engage into the internal thread of the main tightening means 60. The inner tightening means 70 is configured as a second grub screw.

As shown in the two cross-section views of Fig. 6B and 6C , the inner and main tightening means 60 and 70 are threadedly engaged by means of an inner thread 66 and an outer thread 74, respectively. Upon rotation of the inner tightening means, the inner tightening means can travel through the main tightening means in such a manner that respectively the main tightening means bottom side 62 or the inner tightening means bottom side 72 is the lower or distally projecting part of the tightening means assembly 60/70.

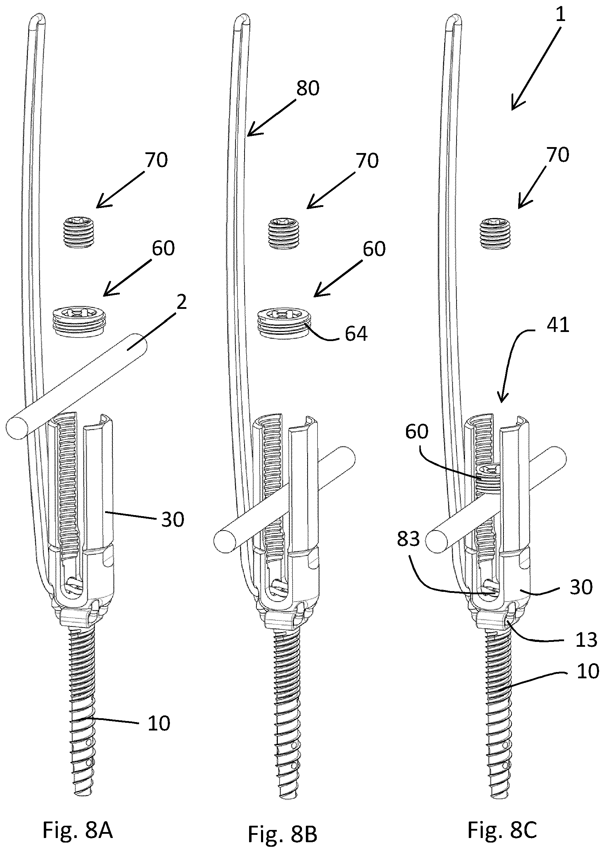

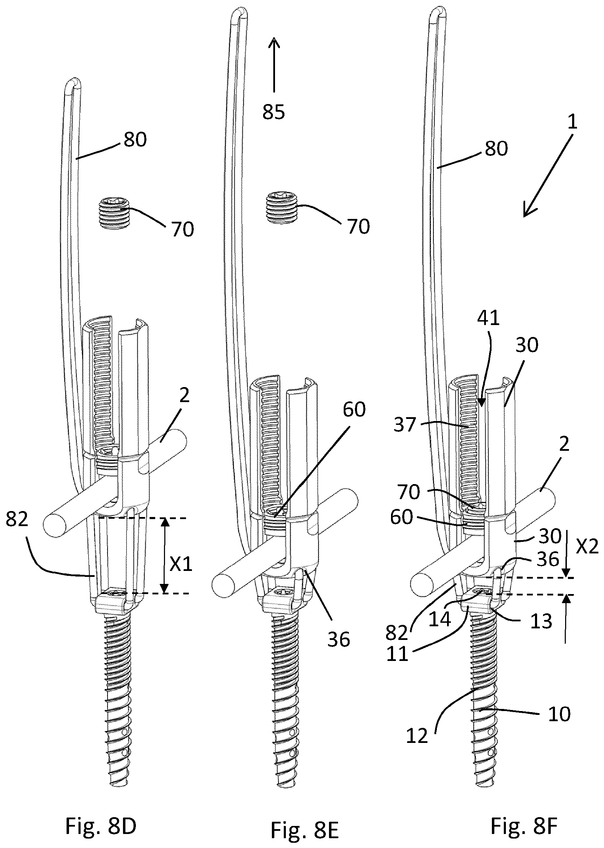

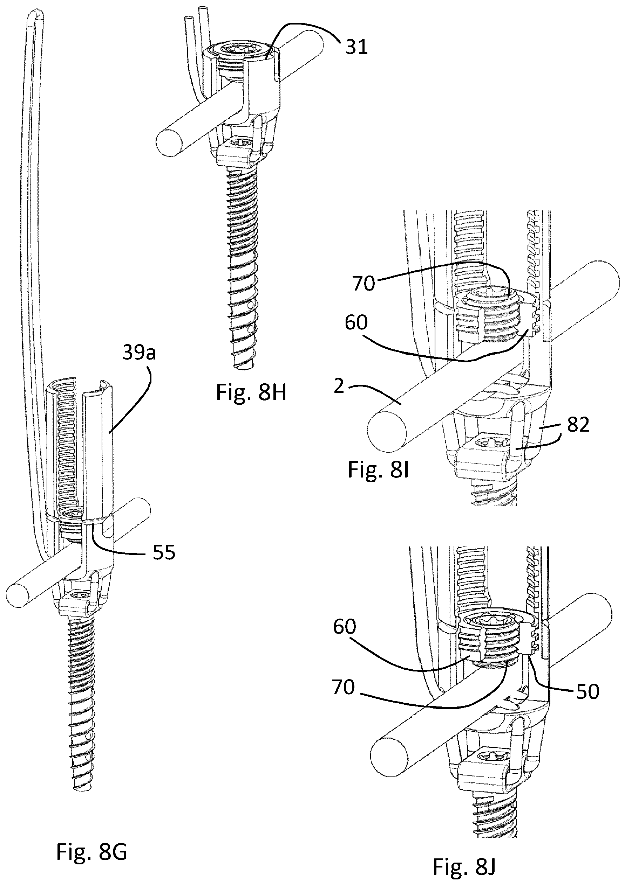

As explained in greater detail for Figs. 8, 9, 11, and 18 to 25, the main and inner tightening means 60, 70 can fulfil different tightening functions. The main tightening means 60 can for example rigidly fixate a spinal rod in an end position, or can hold a rod adjacent to an end position or can simultaneously fixate the rod and the tether in an end position. The inner tightening means can simultaneously fixate the rod and tether, or can specifically only fixate the tether. Hence the main tightening means can function as a spinal rod setting means, a spinal rod fixating means and a combined spinal rod and tether fixating means. Respectively, the inner tightening means can function as a combined rod and tether fixation means, or can function as an isolated tether fixation means.

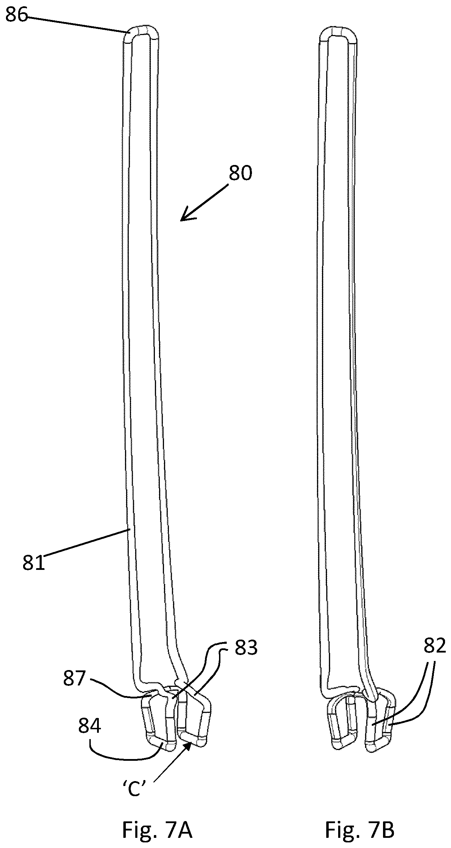

Fig. 7A and Fig. 7B show two different perspective views of the disposition of the flexible elongated member 80 or tether or tether band without the other elements of the spinal bone fastener assembly 1. The flexible elongated member or tether 80 comprises a thin, long elongated flexible body 81 which is sized and shaped to engage into the first and second flexible elongate member retaining means 13 and 36. At least a distance determining portion 82 spans between the proximal end 23 of the bone fastener head portion 11 and the rod receiving head bottom end 32. Reference numeral 83 is related to the to be blocked tether portion disposed over the clamping seat 42 and reference numeral 84 (also marked as 'C') is related to the bone fastener guided portion arranged in the flexible elongate member retaining means 13. Reference numeral 87 is related to the loop portion of the flexible elongated member 80. In other embodiments this loop portion 87 can be replaced by a further long elongated flexible body 81 towards a symmetrically arranged distal coupling end 86.

The flexible elongated member 80 typically is made of woven or braided strands of a surgical suture material such as biocompatible UHMWPE fibres, nylon fibres, Polyester fibres, PET fibres. Alternatively biocompatible metal materials could be used, such as a Stainless Steel, Cobalt-Chromium or Titanium (alloy) cable. Polyaramide, carbon fibres and silk are further alternatives. Furthermore the flexible elongated member 80 has at least one distal coupling end 86 for attachment to an insertion device 140.

Fig. 8A to 8J show sequences of the different steps of mounting of a spinal rod 2 into the spinal bone fastener assembly 1. For illustration purposes the neighbouring pedicle screws and the vertebral bodies in which the bone fastener 10 is fixated are not shown.

Fig. 8A to Fig. 8C shows a sequence of introduction of a spinal rod 2 in the central pocket 41 of a rod receiving head 30 followed by blocking the spinal rod 2 by the main tightening means 60. From Fig. 8A to Fig. 8C the main tightening means 60 is screwed in the internal thread feature 40 of the rod receiving head 30 until the main tightening means 60 abuts against the end seat 38 adjacent to the end of the inner thread. In this example, the distance from the lower surface of the main tightening means 60 to the bottom of the groove in the rod receiving head 30 is larger than the diameter of the spinal rod 2. Therefore, the tether 80 remains free or unclamped, and can still travel through the flexible elongate member retaining means 13 and 36. In other words the spinal rod 2 is not blocking the movement of the tether. Fig. 8D to Fig. 8F show the sequence of reducing a distance X1 between the surface of the head bottom end 31 and the proximal end of the bone fastener head portion 11 towards a distance X2. Fig. 8D shows a first status of the spinal bone fastener assembly 1 in which the bone fastener head portion 11 is spaced at a first distance X1 from the rod receiving head bottom end 32.

By tensioning the tether 80 (in a proximal direction or in the direction of arrow 85) said distance X1 is reduced to a distance X2. In a following step the inner tightening means 70 is assembled and screwed into the main tightening means 60. The inner tightening means travels through the main tightening means 60 and abuts with its bottom side 72 against the spinal rod 2 and so forces the spinal rod towards the clamping seat 42, whilst the tether is arranged between the spinal rod and the clamping seat. Upon final tightening of the inner tightening means, the spinal rod, tether and clamping seat are blocked against each other and any movement of the tether and spinal rod is inhibited. In other words, the spinal rod 2 blocks the portions 83 of the tether 80. In order to change the spinal bone fastener assembly 1 to a further different distance X2 between the bone fastener head portion 11 and the rod receiving head bottom end 32 the spinal rod 2 and the tether can be loosened through un-screwing the inner tightening means 70 and so allowing the blocked tether portions 83 to move freely again in the flexible elongate retaining means 13, 36.

Fig. 8D and Fig. 8E show maintaining a specific spinal rod 2 position in which the spinal rod can still be moved along its longitudinal direction as well as allowing the strings of tether 80 to be moved. On the other hand Fig. 8F shows a locked configuration. In the locked status, the flexible elongate member or tether 80 is biased against the clamping seat 42 by the spinal rod inhibiting a translation of the flexible elongate member 80 through at least one of the first and second flexible elongate member retaining means 13 or 36.

Fig. 8G and Fig 8H show the final removal of rod tightening means receiving extensions 39a, 39b and the resection of the remaining tether strands.

Fig. 8I shows a detail and partial cross-sectional view of a spinal bone fastener assembly 1 of Fig. 8A. Fig. 8I shows the status as described for Fig. 8E, wherein the internal second rod tightening means 70 is arranged in a position allowing a longitudinal movement of the spinal rod 2 and Fig. 8J shows the status as described for Fig. 8F, wherein the internal second tightening means 70 is blocking the spinal rod 2 and the tether 80.

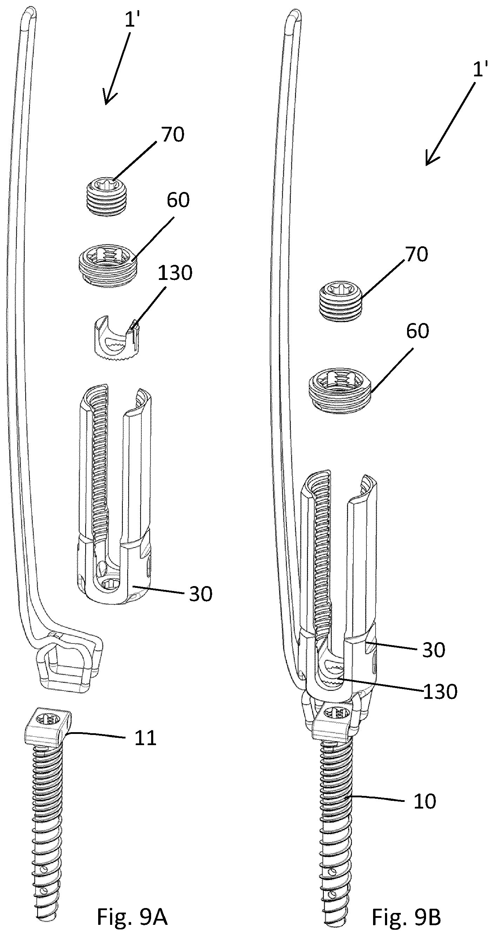

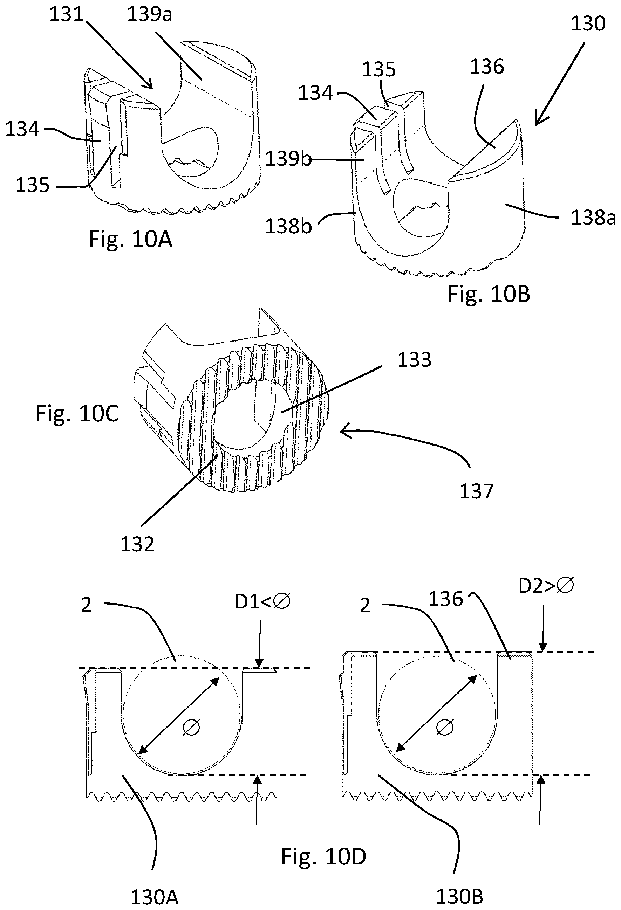

Fig. 9A shows an exploded view of a variant spinal bone fastener assembly 1' similar to the assembly 1 according to Fig. 1A. This variant includes an intermediate inlay 130 configured to hold the spinal rod 2 and clamp the tether 80 into the clamping seat 42. Fig. 9B shows a perspective view of Fig. 9A with the inlay assembled in the rod receiving head 30. Fig. 10A to Fig. 10C show perspective views of the intermediate inlay 130 of Fig. 9A while Fig. 10D shows two side views of two different heights of the inlay for the standard spinal bone fastener assembly application and a growth guiding application. Within the growth guiding application, the spinal rod 2 remains freely movable along the longitudinal axis, whilst the tether 80 is clamped and blocked.

A variant spinal bone fastener assembly 1' has a rod receiving head 30 with an U-shaped groove creating the central pocket 41 allowing a spinal rod 2 to be positioned in transversal direction. The difference between the spinal bone fastener assemblies 1 and 1' is the space and recess provided inside and within the rod receiving head 30 to accommodate an intermediate inlay 130. The intermediate inlay 130 comprises a second rod receiving opening 131 and on the bottom side 137 a clamping surface 132. Furthermore the intermediate inlay 130 comprises a central screwdriver passage 133 allowing to engage a screwdriver into the first drive 14 of bone fastener 10 through the rod receiving head. The central screwdriver passage 133 also provides the possibility to introduce the bone-cement injecting device into the bone fastener head 11. The intermediate inlay 130 moreover has a U-shape and two leg portions 138a and 138b having a top surface 136. The proximally directed leg portions 138a, 138b are formed by the second rod receiving opening 131, which defines the inner wall 139a and 139b of the leg portions 138a, 138b. The leg portions are configured to at least partly encompass the spinal rod. In this example, one of the leg portions has two vertically (top to bottom) oriented side slits 135 forming a central locking element 134. The central locking element 134 is configured as a compliant snap mechanism which is sized and shaped to engage into a corresponding recess in the rod receiving head. This engagement inhibits inadvertent release of the intermediate inlay during implantation or handling of the spinal bone fastener assembly. The intermediate inlay 130 or the clamping seat 42 may comprises a rough structure such as grooves or another macro/micro roughness to engage with the flexible elongated member 80 and so provide an increased friction.

The intermediate inlay 130A for the tether screw application and the intermediate inlay 130B for the growth guiding application have an internal groove or rod receiving opening 131 having a transversal diameter or width sufficient to accommodate for the diameter of a spinal rod 2. The difference between the two inlays 130A and 130B is the height difference D1 and D2 between the top surface 136 of the inlay 130A or 130B and the bottom surface of the groove / second rod receiving opening 131, respectively.

Within the inlay 130A, the distance D1 is smaller than the diameter of the spinal rod 2. Therefore, when the inner tightening means 70 is screwed into the main tightening means then it abuts against the top of the spinal rod and blocks it.

Within the inlay 130B, the distance D2 is larger than the diameter of the spinal rod 2. Therefore, when the inner tightening means 70 is screwed into the main tightening means, it will abut against the top surface 136 and there will remain a small gap between the top of the spinal rod and the inner tightening means bottom side 72 and hence the spinal rod can still move along its longitudinal direction.

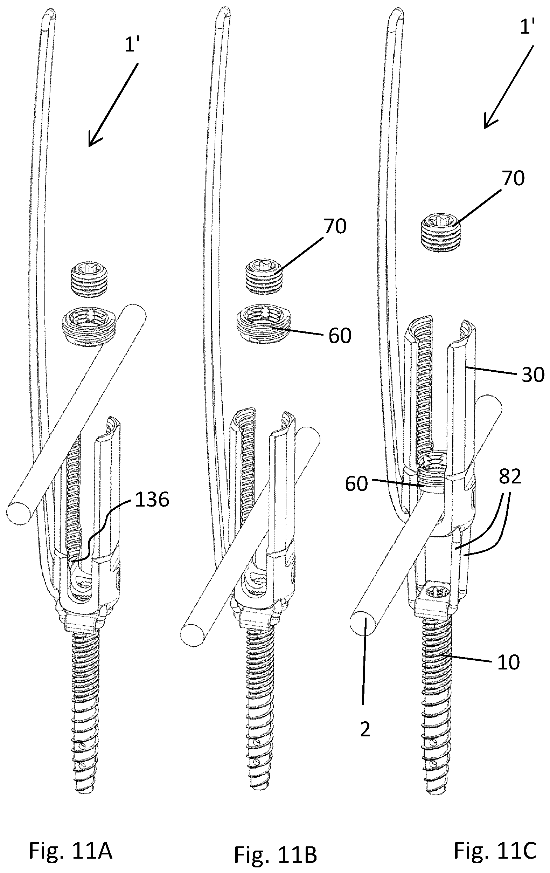

Fig. 11A to 11H show a plurality of perspective views of the introduction and fixation of a spinal rod 2 in the variant spinal bone fastener assembly 1' with the intermediate inlay 130. Fig. 11A shows the insertion of the rod. Fig. 11B shows the rod 2 in a start position. Fig. 11C shows the insertion of the main tightening means 60. Upon engagement of the main tightening means with the spinal rod, the rod receiving head 30 is forced to travel towards the spinal rod 2, since the rod 2 is already positioned near the spine and fixated in neighbouring pedicles. Therefore the distance determining portions 82 become longer, since on the other side, the bone fastener 10 is already screwed in the associated pedicle.

Here, the main tightening means 60 is screwed in the inner thread of the head 30 which ends above the surface 136. In other words, the main tightening means 60 abuts in the head 30 before coming into contact with the spinal rod 2.

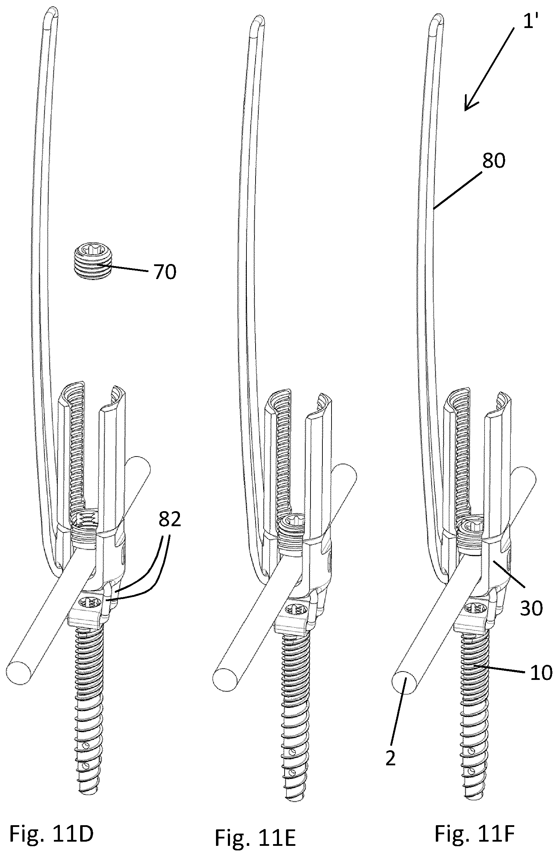

The distance determined by the portions 82 of the tether of Fig. 11C can be adapted through tensioning of the tether 80. As shown in Fig. 11D the distance 82 is shortened / reduced. Fig. 11E shows the insertion of the inner tightening means 70 and Fig. 11F shows the final tightening of the inner tightening means 70 against the rod 2 which presses against the intermediate inlay 130 and which as a result is blocking the tether 80.

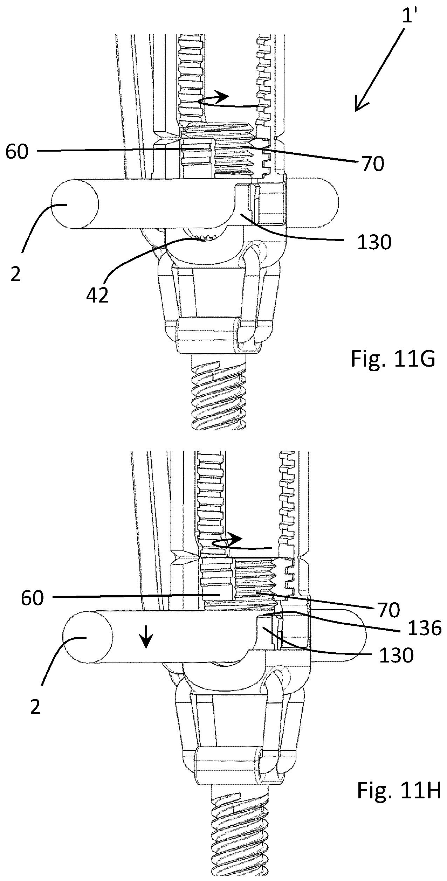

Fig. 11G shows the unclamped clamping mechanism and Fig. 11H shows the clamped clamping mechanism according to the fastener assembly 1' with an intermediate inlay 130. The inner tightening means 70 is advanced forward through the main tightening means 60 and clamps the tether 80 by engagement against the spinal rod 2.

In the configuration as shown in Fig. 11G the spinal rod 2 can still move between the main tightening means bottom side 62 and the intermediate inlay 130. The main tightening means 60 is abutting in the rod receiving head 30 against an end seat 38, and the intermediate inlay 130 is spaced from the clamping seat 42 in such a manner that the tether 80 and spinal rod 2 are unclamped.

Fig. 11 H shows the inner tightening means bottom side 72 engaging against the spinal rod which is captured in the intermediate inlay 130. Upon advancement of the inner tightening means 70 towards and into the end position, indicated by the rotating arrow, the generated clamping force of the thread mechanism is transferred to the spinal rod 2, indicated by the arrow in spinal rod 2, to the intermediate inlay 130 and finally to the tether 80 which is arranged between inner tightening means bottom side 72 and the clamping seat 42, which opposes the clamping force.

In this example the main tightening means 60 acts as a rod setting means, and the inner tightening means 70 act as a rod and tether fixation means. In other words, upon tightening of the inner tightening means into the end position, the tether and the spinal rod are blocked simultaneously.

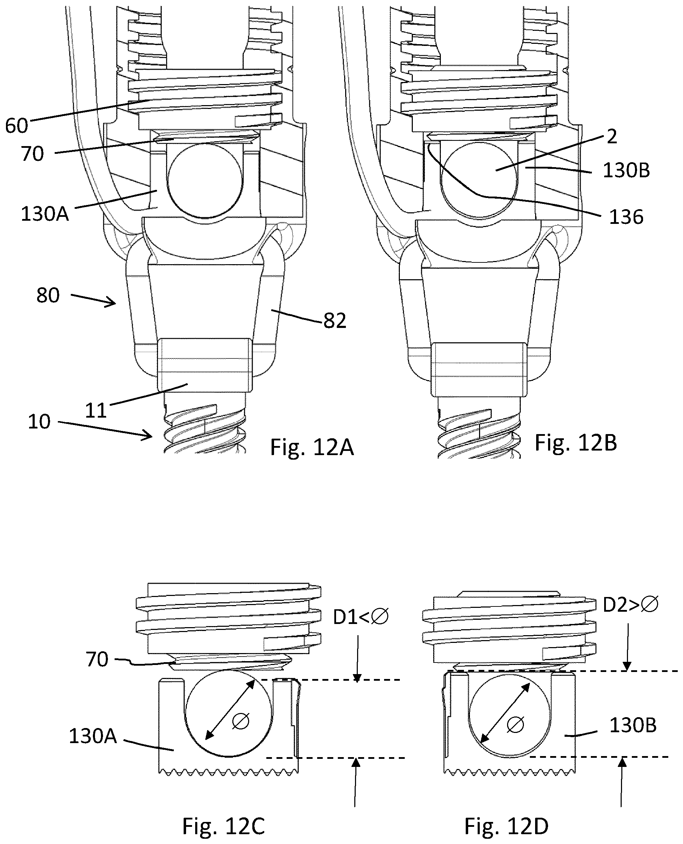

Fig. 12A to Fig. 12D show a plurality of perspective views of the introduction and fixation of a spinal rod 2 in the variant spinal bone fastener assembly 1' with two different intermediate inlays 130A and 130B. Fig. 12A and 12C show the variant of inlay 130A wherein upon tightening of the inner tightening means 70 the tether 80 and the spinal rod 2 are blocked. Fig. 12B and 12D show the variant of Inlay 130B wherein upon tightening of the inner tightening means 70 only the tether 80 is blocked. The spinal rod 2 remains mobile in the growth-guiding spinal bone fastener assembly variant.

The difference between the two inlays 130A and 130B is based on the distance D1 or D2 being smaller or larger than the diameter ∅ of the spinal rod 2, respectively.

Fig. 13A to 13D show an assembly of the insertion device 140 to the spinal bone fastener assembly 1. The combination of an assembly of the insertion device 140 with a spinal bone fastener assembly 1 (or 1') creates a kit 300

Spinal posterior surgeries often are performed in a percutaneous and/or trans-muscular manner. The surgery site is near delicate soft tissue structures such as nerves and vascular structures, and therefore a high safety is required when instruments are used for specific surgical steps. Hence typically, spinal instrument systems comprise an outer, cannulated instrument that guides other instruments within its central cannulation and so reduces the risk of inadvertently damage to other structures.

Hence, the insertion device 10 as depicted in Figs. 13A to 13G is an elongate device that can hold the spinal bone fastener assembly 1 and facilitates several purposes, namely;
- providing an extension of the spinal bone fastener assembly for manipulation of the spinal bone fastener assembly by the operating surgeon
- safe guiding of support instruments such as a first and second screwdriver,
- safe guiding of an assembly/disassembly key for attachment and dis-attachment of the spinal bone fastener assembly,
- a controlled tensioning of the tether.

Fig. 13E to Fig. 13G show the assembly of the first screwdriver 160 into the insertion device 140 and into the spinal bone fastener assembly 1, respectively through the rod receiving head 30, through the first drive shaped aperture 49 and into the complementary sized and shaped first drive 14 of the bone fastener 10. The screwdriver 160 has a hexa-lobe (torx) first working head 162 and moreover a first handle 161. In this example the first working head couples the first drive shaped aperture 49 of the rod receiving head 30 with first drive 14 of the bone fastener 10. As a result, upon rotation of the first screwdriver 160 to implant the bone fastener 10 into the target bone, the entire spinal bone fastener assembly 1 including assembled insertion devices is rotated.

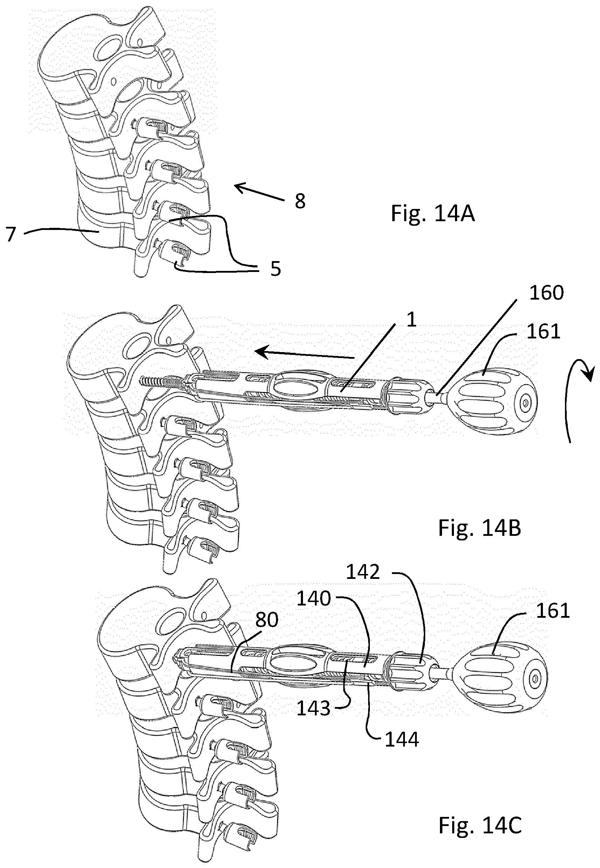

Fig. 14A to Fig. 14Y show the surgical steps for the implantation of the spinal bone fastener assembly 1 in a spine 8 of a patient.

Fig. 14A shows an exemplary section of the spine, namely five vertebral segments 7 of a spine or spinal column 8. Four standard pedicle screws 5 are placed in the lower levels of the spine 8. Fig. 14B shows the spinal bone fastener assembly 1, mounted within the insertion device 140, being introduced through a pedicle in the fifth, here upper most shown vertebral body. For illustration purposes, only a small section of the spine is shown, more specific a thoracic section. Further vertebral bodies are following the upper and lower vertebral body. It is to be noted that in the shown example a proximal junctional kyphosis prevention scenario is shown. Similar steps may apply for a distal junctional kyphosis scenario. Moreover the pedicle screws and spinal rod construct may be longer and involve more vertebral bodies or segments. It is to be noted that for illustration purposes the surgical steps are only shown at one side of the spine.

Fig. 14B shows the insertion device 140 being pre-mounted with the spinal bone fastener assembly 1 and the first screwdriver 160 is turned for screwing the bone fastener 10 into the pedicle bone. Fig. 14C shows an end position of the bone fastener 10, namely the bone fastener head portion 11 is arranged adjacent to the outer surface of the target pedicle bone and the threaded elongated shaft 12 is submerged into the pedicle bone and vertebral body. The oversized bone fastener head portion forms a mechanical stop to inhibit from too deep insertion and unwanted blocking of the tether 80 which is disposed there in. It is noted that the tether 80 is arranged alongside the insertion device 140. The insertion device 140 has a tensioning knob 142 which is connected to tensioning mechanism 148 configured as an internal spindle construct 143 on which a tether holding hook 144 is disposed. Upon rotation of the tensioning knob, the holding hook is actuated to translate along the longitudinal axis of the insertion device 140 and so will be tensioning the tether 80. In this example, clockwise turning will tension the tether. The holding hook 144 can be provided on both sides of the device 140 when there are (as will be discussed later) two tethers 80 provided symmetrically on both sides of the head 30. It is not necessary that the tethers 80 are provided symmetrically.

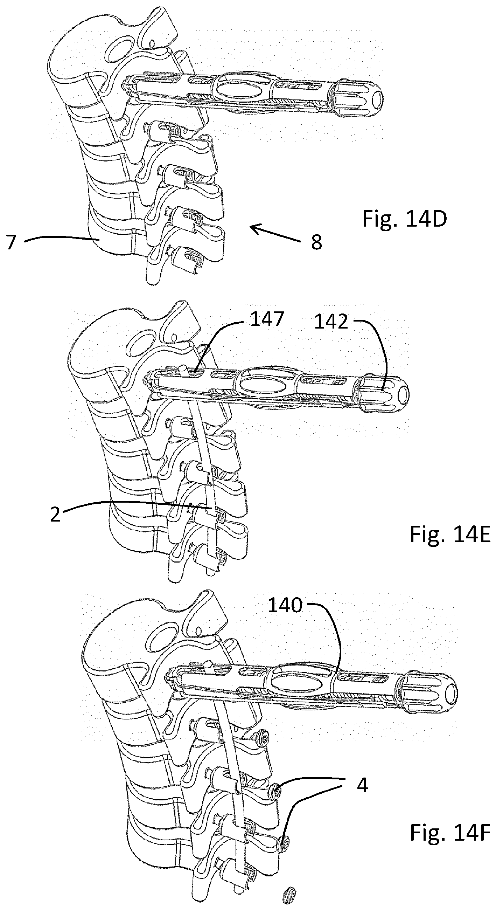

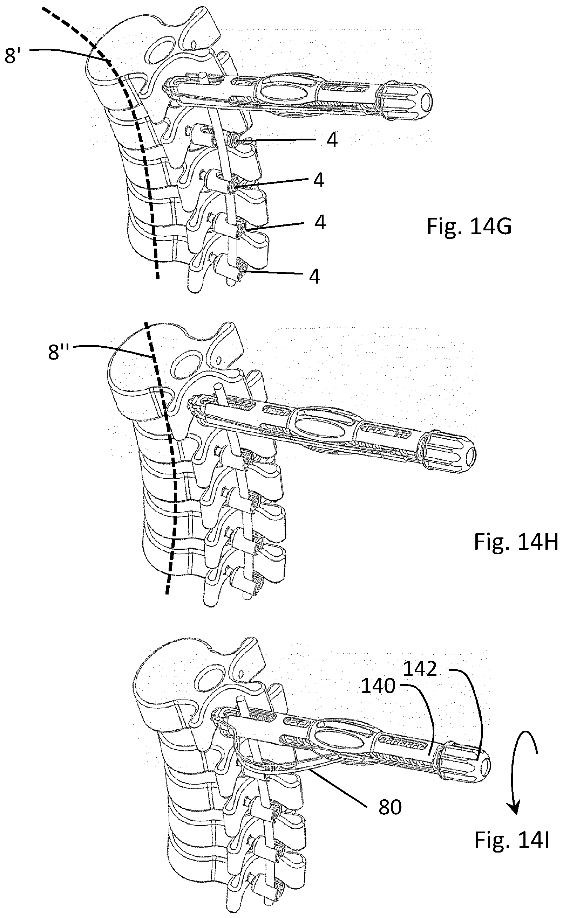

Fig. 14D shows the screwdriver 160 having been removed through retracting it by pulling back. Fig 14E shows the insertion of the spinal rod 2 in the head of the pedicle screws 5 and through the first rod receiving opening 33 of the spinal bone fastener assembly 1 and furthermore through a parallel slit of the insertion device 140. As depicted the first rod receiving opening 33 and the parallel slit at least partly overlap. The next step concerns the fixation of the spinal rod 2 in the standard pedicle screws 5 with setscrews 4, called standard Fig. 14A and Fig. 14G.

Fig. 14F and 14G show the placement of the setscrews 4 of the standard pedicle screws. The spine 8' is being straightened as shown by the dashed line when the setscrews are tightened as shown in Fig. 14H. Fig. 14I shows the release of the tether 80 which provides mobility to the rod receiving head 30. Turning tensioning knob 142 of the insertion device 140 counter-clockwise relaxes the tether 80.

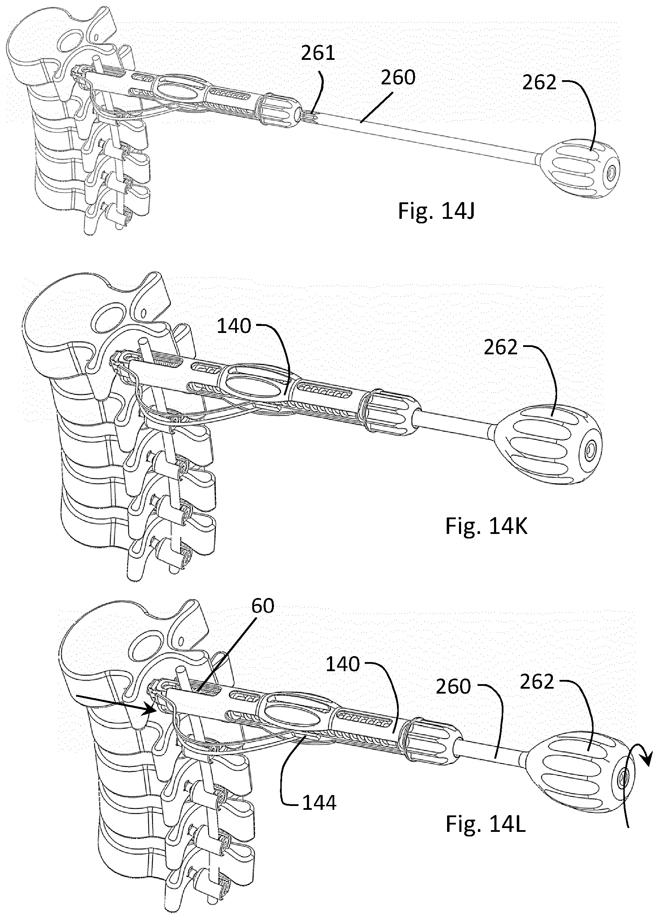

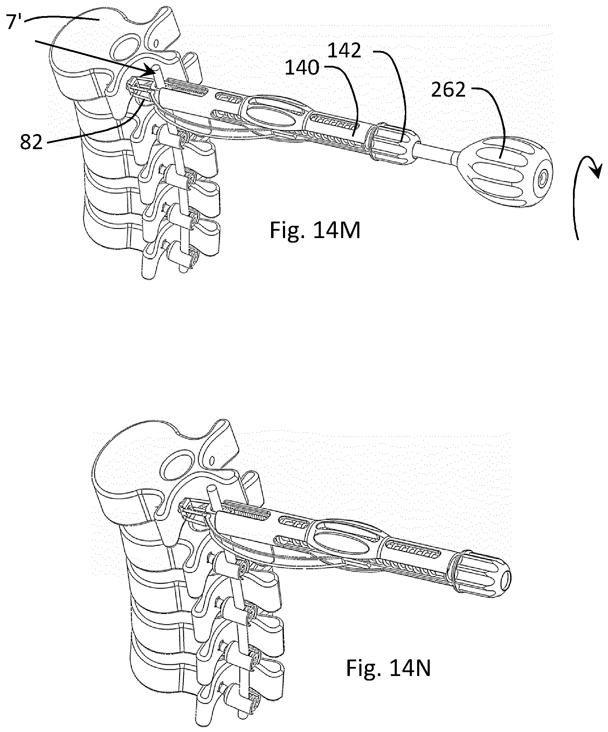

Fig. 14J shows the insertion of a second screwdriver 260 comprising a second working head 261 and a second handle 262. The working head 261 is advanced forward in the insertion device 140 and engaged into the main tightening means 60 as shown in Fig. 14K. Via turning of the second screwdriver 260 as shown in Fig. 14L in the clockwise direction, the main tightening means 60 is tightened and engaged against the spinal rod 2. Upon engagement and further tightening, the rod receiving head 30 is forced to move towards the spinal rod 2 while the vertebral segment 7' in question stays at its position and the distance determining portion 82 of the tether 80 becomes longer as shown in Fig. 14M.

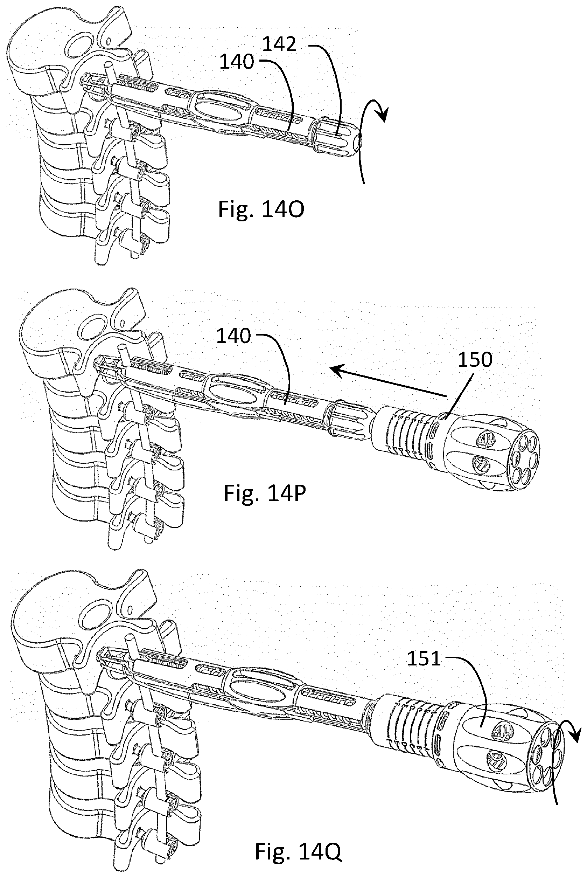

Fig. 14N shows the situation in which the second screwdriver 260 is removed. The following step is shown in connection with Fig. 14O. Pre-tensioning of the tether 80 by turning of the spindle construct 143 in the device 140 in opposite direction as shown in Fig. 14I the hook 144 is travelling towards the knob 142 straightening the tether 80.

Fig. 14P shows the positioning of a force measuring device 150 on the insertion device 140. The force measuring device 150 is coupled with the tensioning knob 142 of the insertion device 140 as shown in Fig. 14Q. Upon rotation of the turning knob 151 of the force measuring device 150, the applied torque will be transferred to the spindle construct and create tension on the tether. In this example the force measuring device provides feedback on the actual tension in the tether.

Knowledge of the applied tension is of importance; too much tension as well as too little tension could make the spinal bone fastener assembly to fail to provide the targeted gradual stress transition from the fused spine to the mobile spine.

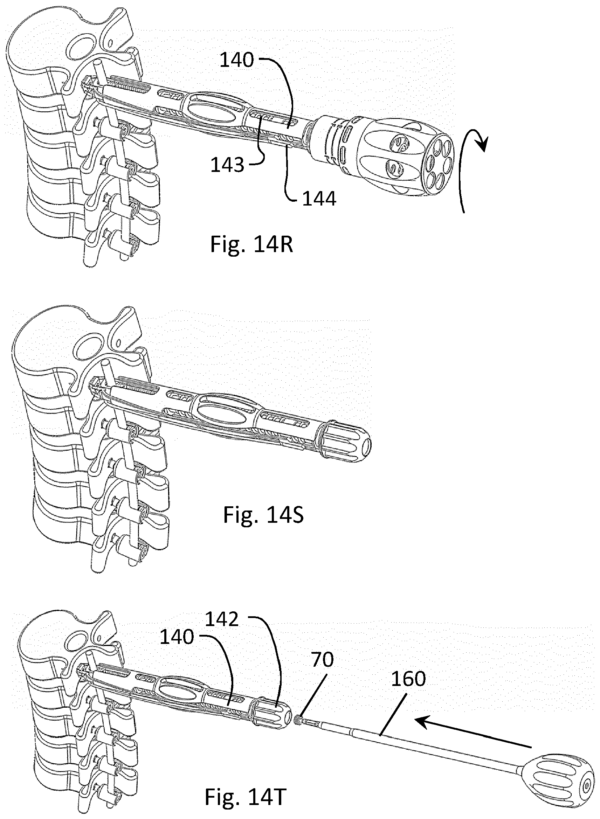

Fig. 14R shows the tightening process of the tether 80 to the appropriate tension using the force measuring device 150. The spindle construct 143 of the insertion device 140 transforms the applied torque into a longitudinal movement of the tether holding hook 144 and thus in a tensional force on the tether 80. Fig 14S just shows the removal of the force measuring device after the correct predetermined tension is reached.

The next step is shown in Fig. 14T. Preferably, it is the same first screwdriver 160 inserted in the insertion device 140 to avoid too many instruments, but it can be a different too. As described earlier, the insertion device 140 and all its elements comprise a central passage which is oversized in relation to other instruments such as the first and second screwdriver 160, 260 and the inner tightening means 70. Therefore the inner tightening means can be engaged into the main tightening means using first screwdriver 160,

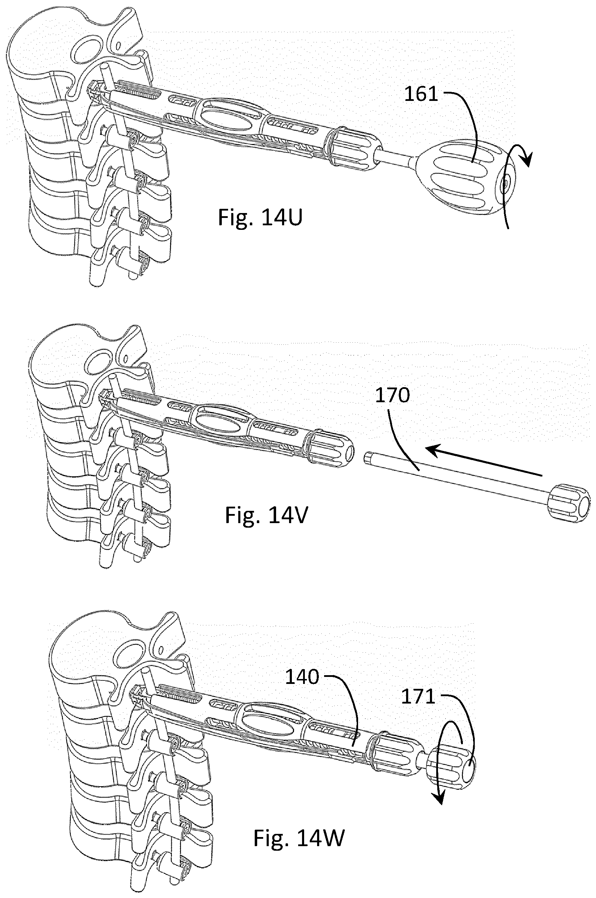

Fig. 14U shows turning of the screwdriver head 161 in a clockwise rotation of the screwdriver 160 which allows the inner tightening means 70 to be tightened into the main tightening means 60 and the whole system is blocked. This step is also shown in Fig. 11G and 11H.

After removal of the first screwdriver 160, Fig. 14V shows introduction of an assembly/disassembly key 170. The assembly/disassembly key 170 is inserted into the insertion device 140. As shown in Fig. 14W, by counter clockwise rotation of the turning knob of the assembly/disassembly key 170 the insertion device 140 is decoupled from the rod receiving head 30.

The shaft of the assembly/disassembly key 170 is advanced in the insertion device 140 and engaged into a rotatably coupled locking bolt 146 of the insertion device. Upon rotation of the locking bolt 146, by means of the assembly/disassembly key 170, the locking bolt 146 is threaded into the rod receiving head 30, more specifically, made to engage into the internal thread of rod tightening means receiving extensions 39a, 39b.

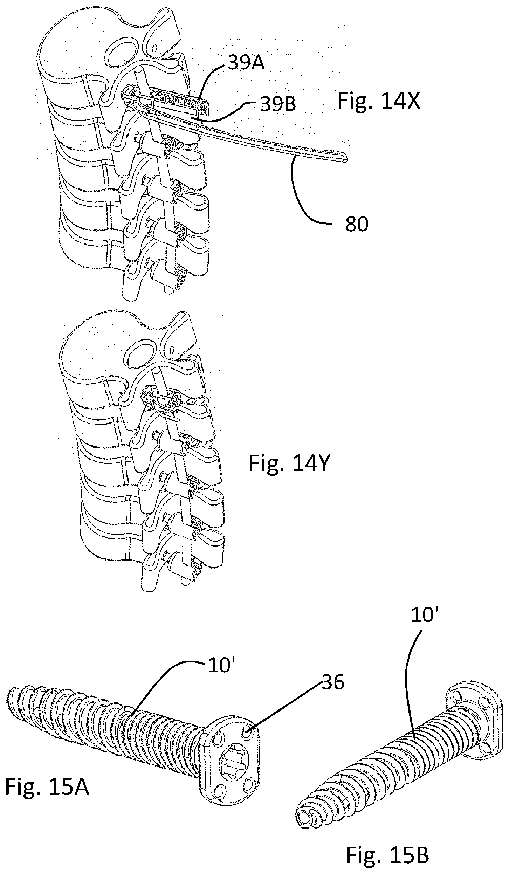

Fig. 14X shows the pedicle screw and posterior rod construct after removal of the insertion device 140 including the remaining loop of the tether 80 and the two side extensions 39a and 39b.

Fig. 14Y shows the situation after the next working steps on the spinal bone fastener assembly 1, i.e. the extensions 39a and 39b are cut or broken off at breaking grooves at the free end of the rod receiving head 30 and the remaining tether of the tether loop 80 strand is cut off.

Fig. 14A to Fig. 14Y show four standard pedicle screws 5 being placed in the lower levels of the spine 8 and one bone fastener assembly 1 being introduced through a pedicle in the fifth, here upper most shown vertebral body. Alternatively two or more bone fastener assemblies 1 may be placed at the end of a spinal rod construct. Is this case it is of importance that the tether 80 are tensioned in such a manner that both tethers are tight. It is to be inhibited that an overtightening of one tether in relation to another tether unloads one of the bone fastener assemblies. In a preferred manner, the spinal bone fastener assembly arranged at the end of the spinal rod is tensioned less than the spinal bone fastener assembly arranged more towards the middle of the spinal rod.

In one other embodiment the spinal bone fastener arranged at the end of the rod is tensioned with a force of 0-40 Newton and the spinal bone fastener arranged towards the middle of the spinal rod is tensioned with a force of 40-100 Newton.

Fig. 15A and Fig. 15B show perspective views of a variant of bone fastener 10'. This bone fastener variant 10' has proximal-distal flexible elongate member retaining means 13, i.e. directed in the longitudinal direction of the bone fastener 10. The flexible elongate member retaining means 13 are arranged in a substantially regular or symmetric manner around the elongated shaft 12 to provide for a uniform stress distribution in the target bone.

All previous described embodiments of the spinal bone fastener assembly 1, were described in the context of a U-shaped rod receiving head 30 which is extending away from the head bottom end 32 and is open towards the insertion device 140. Other variants are possible without departing from the previous described surgical method of implantation of the spinal bone fastener assembly 1 as shown in Fig. 14A to 14X.

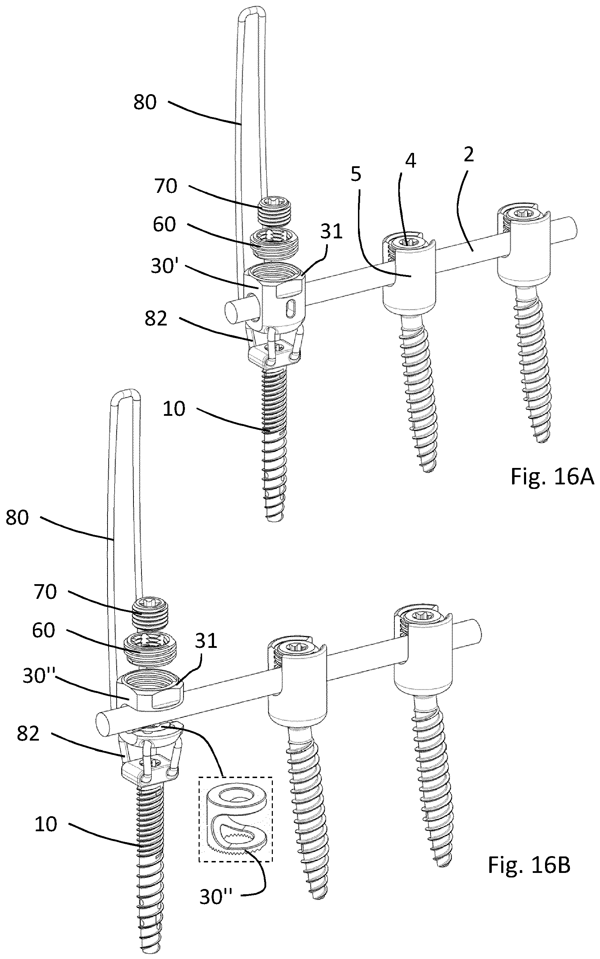

Fig. 16A shows a perspective view a front-loading rod receiving head 30'. The variant with front-loading head 30' most practically is used at the end of a construct, due to the rod receiving opening having a closed configuration. The rod receiving opening 33' is sized and shaped as a through-bores for the spinal rod 2. When used in the middle of a construct, the spinal rod has to translate through the rod receiving head, before engaging the spinal rod in other standard pedicle screws.

Fig. 16B shows a perspective view a side-loading rod receiving head 30". The variant with side-loading rod receiving head 30" can be used at any place of a construct, since the head is not closed and the U-shaped opening is oriented substantially orthogonally to the longitudinal direction of the rod receiving head 30". As depicted in the detail view section of Fig. 16B, the side loading rod receiving head may comprise a corresponding side loading intermediate inlay 130'.

However, it is not possible to use the inlay 130A or 130B as shown above. It is however possible to use a similar inlay with a shape in L and the inner screw 70 is then pushing only on one side. In Fig. 16B an inlay is shown being u-shaped, wherein the second rod receiving opening 131 is directed towards the side.

Fig, 17A and Fig. 17B show a variant with double rod receiving head 30D to receive two spinal rods 2. Hence the rod receiving head comprises a first rod receiving opening 33 and a neighbouring rod receiving opening 53. Fig. 17A shows the variant with extensions 39a, 39b and 39c in place. The middle extension 39c has two faces towards the rod receiving opening 33 and 53, while the inner thread acts as rod setting or tightening mechanism. Fig. 17B shows the embodiment of Fig. 17A with broken-off extensions 39a, 39b and 39c. It is noted that the first rod receiving opening 33 has an main tightening means 60 and an inner tightening means 70 fixing the double head 30D on the spinal rod 2 as well as blocking the tether 80 in place (still to be cut) while the neighbouring rod receiving opening 53 comprises a single third tightening means or standard setscrew 60".

Spinal bone fastener assemblies with a double rod receiving head are used to for example extend an existing or in-situ system, and/or to provide a transfer to a differently sized rod, and/or to provide higher rigidity. A double rod receiving head can comprise 2 parallel rods and therefore can provide for example a twice as high stiffness.

Fig 18A to 18E show yet another variant of the spinal bone fastener assembly 1". The spinal rod locking principle and tether locking principle of this variant will allow to block the tether 80 and the spinal rod in an individual manner 2. In other words, the clamping force which is generated by the main tightening means solely holds the rod 2 in place and respectively the clamping force generated by the inner tightening means solely blocks the tether 80. As explained in greater detail later, the variant of Fig. 18 moreover comprises an alternative looping sequence or deposition of the tether through and into the second flexible elongate member retaining means of the rod reiving head 30.

Fig. 18A shows an orthogonal side-view of the spinal bone fastener assembly 1" including a cutting plane line at the level of one arrangement of the second flexible elongate member retaining means of the rod reiving head. Fig. 18B shows the corresponding section view, in which only the rod receiving head is sectioned.

The perspective views 18C to 18H depict the spinal bone fastener assembly 1" including the sectioned rod receiving head as described for Fig. 18B. The detail views of Fig. 18D and 18E show the rod receiving head in a non-sectioned manner.

In this example, a similar arrangement of second flexible elongate member retaining means is located at the opposite side of the central plane of the spinal bone fastener assembly.

Fig. 18C shows an initial surgical step wherein the spinal rod is introduced into the rod receiving head 30. In a following step as shown in Fig. 18D, the main tightening means 60 is assembled and screwed towards the spinal rod 2 for engagement therewith. In a next step as shown in Fig. 18E the main tightening means 60 is advanced further by means of screwing and forces the rod receiving head 30 to travel towards the spinal rod 2. During this process the tether portion which is arranged between the rod receiving head bottom end 32 and the proximal end of the bone fastener 10 elongates. The tether 80 travels through the first and second flexible elongate member retaining means 13, 36. As shown in greater detail in Figs. 19, the rod will travel as far to seat against the rod receiving opening end 100 and therefore is directly clamped by the rod receiving head and the main tightening means bottom side 62.

Referring to Fig. 18F, the tightening of the tether is shown. Upon applying tension to the tether, the distance between the rod receiving head bottom end 32 and the proximal end of the bone fastener 10 is reduced.

Fig. 18G shows the engagement of the inner tightening means 70 into the main tightening means 60. The inner tightening means is screwed towards its end position as shown in Fig. 18H. The inner tightening means engages against the intermediate inlay 130, and clamps the tether 80.

Figs. 19A and 19B show details of the tether clamping step and Fig. 19C shows the main tightening means in greater detail. For visualization purposes the rod receiving head 30 and the main tightening means 70 are depicted in a sectioned manner. Fig. 19A shows the spinal rod already being clamped by the rod receiving opening end 100 of the rod receiving head 30 and the main tightening means bottom side 62. The intermediate inlay top surface 136 projects into the threaded throughbore 67 and ends above the most upper surface of the spinal rod 2. The inner tightening means is engaged into the main tightening means and is advanced to touch the intermediate inlay top surface 136. In this instance the bottom side 137 is spaced from the clamping seat 42 and the tether 80 therefore is unclamped. The loop portion 87 is shown on the left hand side and the returning spanning portions 82 are then passing through the passage or flexible elongate member retaining means 36. The blocked tether portion 83 of other embodiments is here realized at the blocked tether portion 183 where according to the black arrow the intermediate inlay 130 is pushed downwards and blocks the tether 80 at this position of the clamping seat.

Fig. 19B shows the next step, in which upon advancement of the inner tightening means the intermediate inlay is forced towards the clamping seat 42, and clamps the tether 80. It is to be noted that in the end position as shown in Fig. 19B, a space is present between the spinal rod 2 and the inner tightening means bottom side 72.

In this example the main tightening means 60 acts as an isolated rod fixation means, and the inner tightening means 70 acts as an isolated tether fixation means.

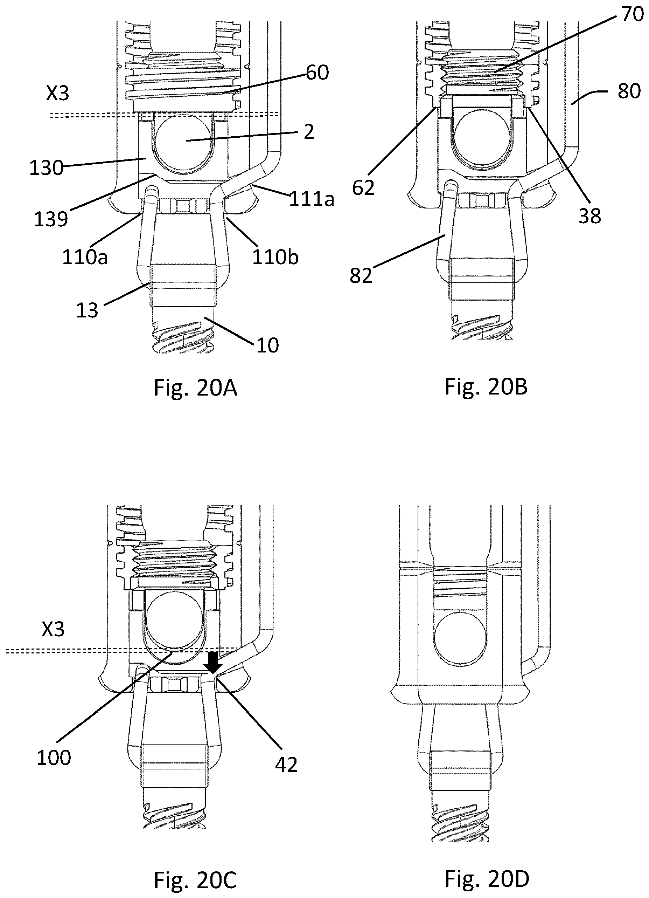

The advantage of the variant of Figs. 18 and 19 is that the tether 80, which is made of a softer material such as artificial fibers, is not being an intermediate force transferring element for the spinal rod clamping process. The spinal rod 2 is directly clamped by hard materials and therefore a reduced risk of rod-slippage is achieved. One further advantage will be described for Fig. 20.

Fig. 19C shows the variant main tightening means 60' in greater detail. The first main body of the main tightening means 60' comprises a stepped section which divides the first main body 61 in a standard diameter portion 68 and a reduced diameter portion 69 located adjacent to the bottom side 62. The outer circumference or periphery of the reduced diameter portion has a smaller diameter than the inner circumference or periphery of the end seat 38. As shown in Fig. 19A, the main tightening means bottom side 62 will bias the rod 2 against the rod receiving opening end 100, before the threadedly engaged outer diameter portion will bias against the end seat 38.

Figs. 20A to 20D show another variant of the spinal bone fastener assembly as described for Figs. 18 and 19. This variant comprises a growth guiding locking principle in which only the tether 80 is clamped and blocked. For visualization purposes the rod receiving head (Fig 20A-20C) and the main tightening means (Fig 20A-20B) are depicted in a sectioned manner.

Fig. 20A and 20B shows the spinal rod 2 being engaged in the rod receiving opening 33. The main tightening means 60 is biased against the end seat 38, and securely locked in this position. As shown, the distance X3 between the main tightening means bottom side 62 and the rod receiving opening end 100 is minimally larger than the spinal rod diameter and hence the main tightening means 60 acts as a rod setting means and the spinal rod 2 remains mobile along its longitudinal axis in relation to the rod receiving head 30.

It is to be noted that in this status the intermediate inlay 130 is spaced from the clamping seat 42 and therefore the tether 80 is unclamped.

Fig. 20C shows the clamping step. The inner tightening means 70 is screwed further into the main tightening means 60, and forces the intermediate inlay 130 to towards the clamping seat 42. As a result, the tether portion which is arranged between them, is clamped. The inner tightening means 70 acts tether fixation means.

It is to be noted that the tether arrangement through the first and second flexible elongate member retaining means differs from the arrangement as described for Figs. 7. The rod receiving head comprises six second elongate member retaining means 36 of which four are substantially regularly divided around the centre of the elongated head 30 and extend from the head bottom end 32 into the clamping seat 42 and define bottom passages 110. The other two second elongate member retaining means 36 extend from the central pocket 41 into the outside of the rod receiving head 30 and are arranged adjacent to the clamping seat 42 and define side passages 111. The six second elongate member retaining means 36 are divided in two substantially symmetrically arranged sets of passages.

With focus on one set of passages, the tether 80 is arranged through the first and second elongate member retaining means in the following sequence. Starting inside the central pocket 41, the tether 80 passes through a first bottom passage 110a towards the bone fastener head portion 11, followed by a passing through a first elongate member retaining means 13, followed by a passing through a second bottom passage 110b from the rod receiving head bottom end 32 into the central pocket 42, followed by a passing through an adjacent arranged side passage 111a and exiting this side passage at the outside of the rod receiving head 30.

Referring to the intermediate inlay 130, as shown this variant comprises a stepped clamping surface 132. The stepped shape is formed by a cut-out and defines a clearance 139 which cannot transmit a high clamping force. As a result, a larger clamping force portion is concentrated on the tether portion arranged at the transition area 190 from the bottom passage 110 to the side passage 111.

The use purpose of the previously described spinal bone fastener assembly 1" can be adapted from a fully locked construct into a growth guiding construct by means of selection of main tightening means 60, 60' variant. The advantage of this principle is that this decision can be made intra-operatively after implantation of the bone fastener 10 in the target bone. A further advantage is a logistical or stock keeping units advantage. The preassembled components of the spinal bone fastener assembly 1" for both purposes are the same, namely the bone fastener 10, rod receiving head 30, intermediate inlay 130 and the tether 80.

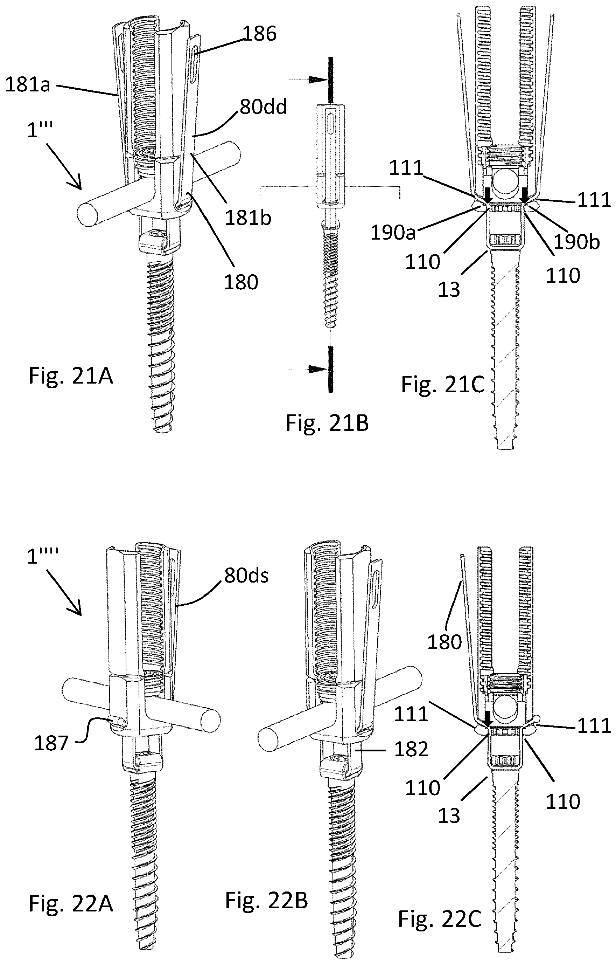

Fig. 21A to 21C show another variant of the spinal bone fastener assembly 1"'. This variant comprises a flexible elongate member 80 configured as a flat tether band 180 with a substantially larger width than thickness. In order to retain the tether band 180 the corresponding first and second flexible member retaining means 13 and 36 have an elongate shape or slot shape.

The example of Fig. 21A to 21C shows the tether band 180 as flexible elongate member having two free strands 181a, 181b arranged alongside of the rod receiving head. At the ends the tether band comprises a distal coupling end 86 configured as an eye 186 for attachment to an insertion device 140 for the tensioning step. As depicted, the second tether retaining means 36 of the rod receiving head 30 are configured as two bottom passages 110 and two oppositely arranged side passages 111.

The tether band 180 is arranged through the first and second elongate member retaining means 13, 36 in the following sequence. Starting alongside the rod receiving head, the tether band 180 passes through a first side passage 111a, followed by passing through the most adjacent first bottom passage 110a, followed by passing towards the bone fastener head portion 11, followed by a passing through a first elongate member retaining means 13, followed by a passing through a second bottom passage 110b from the rod receiving head bottom end 32 into the central pocket 42, followed by a passing through the most adjacent arranged side passage 111b and exiting said side passage at the outside of the rod receiving head 30.

Referring to Fig. 21C, the clamping of the tether band 180 by means of the intermediate inlay 130 is shown. The intermediate inlay 130 is forced towards the clamping seat 42 by means of tightening of the inner tightening means 70 and clamps the tether band 180 at the transition area 190a, 190b from the bottom passage 110 to the side passage 111.

Fig. 22A to 22C show another variant of the spinal bone fastener assembly 1ʺʺ as described for Fig. 21. The example of Fig. 22 shows the tether band 180 having one free strand 181 arranged alongside of the rod receiving head and the tether band 180 having an enlarged stop portion 187 at the opposite side of the rod receiving head 30. The free strand 181 comprises an eye 186 for attachment to the insertion device 140 and to facilitate the tensioning step.

The tether band 180 is arranged through the first and second elongate member retaining means 13, 36 in the following sequence. Starting alongside the rod receiving head, the tether band 180 passes through a first side passage 111a, followed by passing through the most adjacent first bottom passage 110a, followed by passing towards the bone fastener head portion 11, followed by a passing through a first elongate member retaining means 13, followed by a passing through a second bottom passage 110b from the rod receiving head bottom end 32 into the central pocket 42, followed by a passing through the most adjacent arranged side passage 111b and exiting said side passage at the outside of the rod receiving head 30. The enlarged stop portion 183 mates with the outside of the rod receiving head 30 at the first side passage 111a and so inhibits disengagement or unravelling of the tether band 180 out of the first and second elongate member retaining means 13, 36.

Referring to Fig. 22C, the clamping of the tether band 180 by means of the intermediate inlay 130 is shown. The intermediate inlay 130 is forced towards the clamping seat 42 by means of tightening of the inner tightening means 70 and clamps the tether band 180 at the transition area 190 from the bottom passage 110b to the side passage 111b.

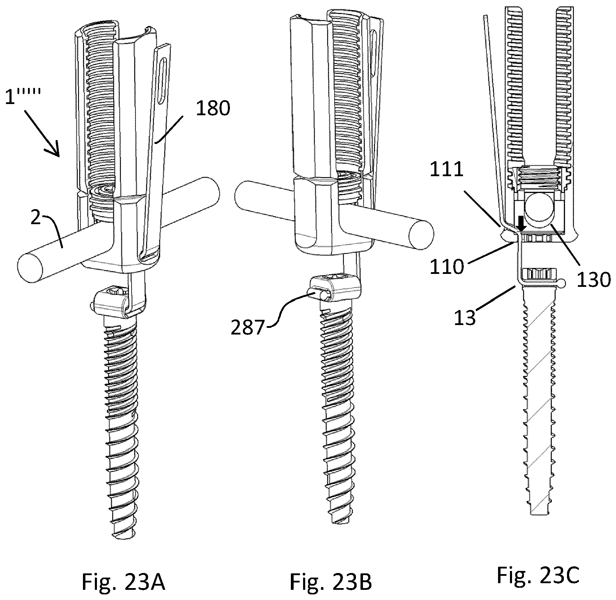

Fig. 23A to 23C show another variant of the spinal bone fastener assembly 1ʺ‴ as described for Fig. 22. The example of Fig. 23 shows the tether band 180 having one free band end portion or strand 181 arranged alongside of the rod receiving head and the tether band 180 having an enlarged stop portion 287 at the opposite side of the bone fastener head portion 11.

The tether band 180 is arranged through the first and second elongate member retaining means 13, 36 in the following sequence. Starting alongside the bone fastener head portion 11, the tether band 180 passes through a first elongate member retaining means 13, followed by a passing through a second bottom passage 110a from the rod receiving head bottom end 32 into the central pocket 42, followed by a passing through an adjacent arranged side passage 111 and exiting said side passage at the outside of the rod receiving head 30. The enlarged stop portion 287 mates with the outside of the bone fastener head portion 11 at the start of the first flexible elongate member retaining means 13 and so inhibits disengagement or unravelling of the tether band 180 out of the first and second elongate member retaining means 13, 36.

Referring to Fig. 23C, the clamping of the tether band 180 by means of the intermediate inlay 130 is shown. The intermediate inlay 130 is forced towards the clamping seat 42 by means of tightening of the inner tightening means 70 and clamps the tether band 180 at the transition area 190 from the bottom passage 110 to the side passage 111. This variant has the disadvantage of an asymmetric loading, but comprises a basic variant of the spinal bone fastener assembly 1.

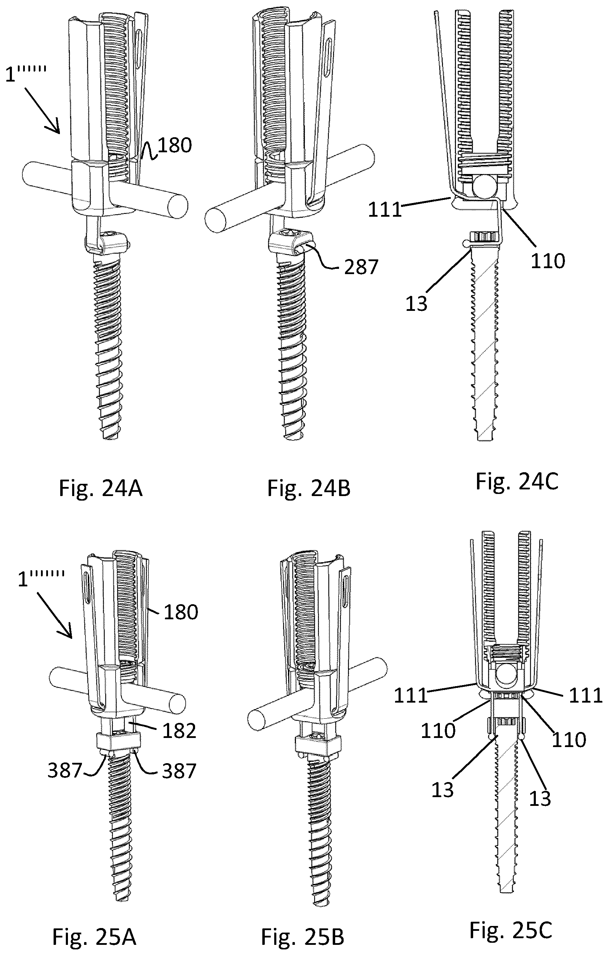

Fig. 24A to 24C show another variant of the spinal bone fastener assembly 1""" as described for Fig. 23. The example of Fig. 24 shows the tether band 180 having one free strand 181 arranged alongside of the rod receiving head and the tether band 180 having an enlarged stop portion 287 at the opposite side of the bone fastener head portion 11.

The tether band 180 is arranged through the first and second elongate member retaining means 13, 36 in the following sequence. Starting alongside the bone fastener head portion 11, the tether band 180 passes through a first elongate member retaining means 13, followed by a passing through a second bottom passage 110a from the rod receiving head bottom end 32 into the central pocket 42, followed by a passing through an opposite arranged side passage 111 and exiting said side passage at the outside of the rod receiving head 30. The tether band 180 spans over a large part of the clamping seat 42 and crosses and intersects with the first rod receiving opening 33.

Referring to Fig. 24C, the clamping of the tether band 180 by means of the spinal rod 2 is shown. The spinal rod is forced towards the clamping seat 42 by means of tightening of an main tightening means and clamps the tether band 180 and the spinal rod 2 into the clamping seat. This variant has the disadvantage of an asymmetric loading, but comprises a basic variant of the spinal bone fastener assembly 1. It is to be noted that this variant does not comprise an inner tightening means 70.

Fig. 25A to 25C show yet another variant of the spinal bone fastener assembly 1‴ʺʺ as shown in Fig. 21. This variant comprises two separate tether bands 180a and 180b. The example of Fig. 23 shows the tether bands 180a, 180b each having one free strand 181 arranged alongside of the rod receiving head and the tether bands 180a, 180b each having an enlarged stop portion 387 at the underside of the bone fastener head portion 11.

The tether bands 180a, 180b are respectively arranged through the first and second elongate member retaining means 13, 36 in the following sequence. Starting at the underside of the bone fastener head portion 11, the tether band 180a passes through a first elongate member retaining means 13a, followed by a passing through a second bottom passage 110a from the rod receiving head bottom end 32 into the central pocket 42, followed by a passing through an adjacent arranged side passage 111a and exiting said side passage at the outside of the rod receiving head 30. The enlarged stop portion 387 mates with the underside of the bone fastener head portion 11 at the start of the first flexible elongate member retaining means 13a and so inhibits disengagement or unravelling of the tether band 180a out of the first and second elongate member retaining means 13, 36. The tether band 180b is arranged in a similar manner through the first and second elongate member retaining means 13b, 36b.

Referring to Fig. 25C, the clamping of the tether band 180 by means of the intermediate inlay 130 is shown. The intermediate inlay 130 is forced towards the clamping seat 42 by means of tightening of the inner tightening means 70 and clamps the tether bands 180a, 180b at the transition areas 190a, 190b from the bottom passages 110a, 110b to the side passages 111a, 111b.

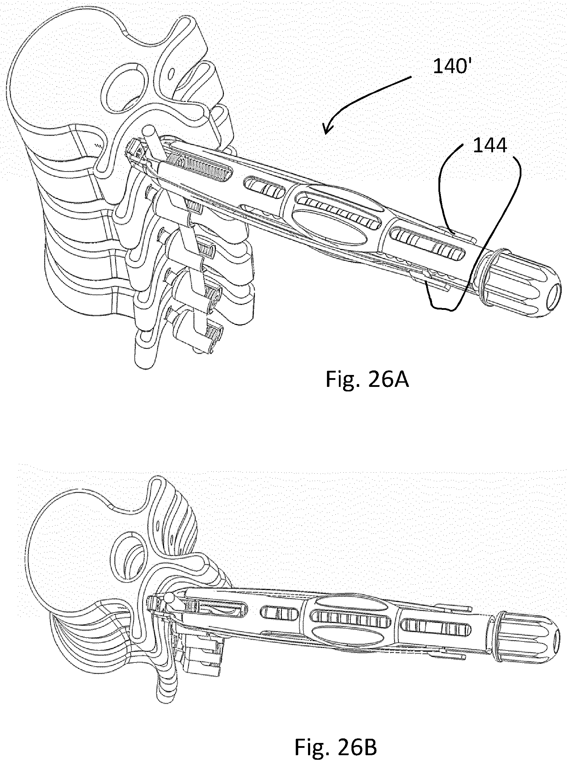

Fig. 26A and 26B show an example insertion device 140' having double sided tether holding hooks 144 for tensioning the example spinal bone fastener assemblies as shown in Figs. 21 and 25, wherein the hooks 144 engage the eyes 186.

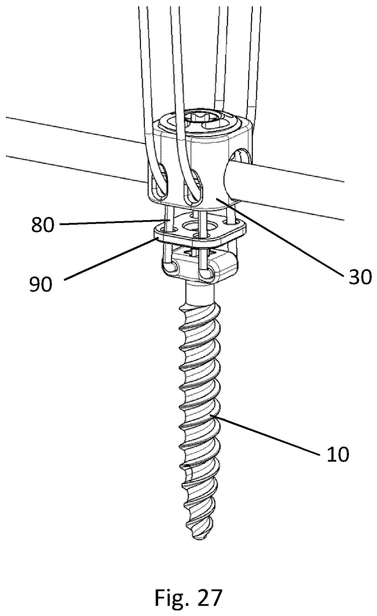

Fig. 27 shows a variant of the spinal bone fastener assembly comprising a spacer 90 between the bone fastener 10 and the rod receiving head 30, provided as an essentially flat disk. The spacer 90 inhibits any collision between bone fastener 10 and the rod receiving head 30 that could cause debris. In this example the flexible elongate member 80 or portions thereof is/are encompassed by the spacer. The spacer 90 may be made of permanent remaining biocompatible plastic material such as PEEK, Polyethylene, etcetera. Alternatively, the spacer 90 may be made of a bio-resorbable material such as poly-lactide, etcetera. The spacer 90 of Fig. 27 has a central hole allowing acccessing the bone fasterner head portion 11 by the rod tightening means 60 or 70. The essentially rectangular spacer 90 with rounded edges further comprises a number of holes, one in each corner accomodating the flexible elongate member 80 passing through them. Such a spacer 90 can be combined with any embodiment of the invention of Fig. 1A to Fig. 26B and e.g. a spacer 90 for the embodiment of Fig. 23A to Fig. 23C comprises one slit as "hole" along one side of the spacer 90 to accomodate the tether band 180. The slit can also be partly open towards the outer edge of the spacer to that the tether band 180 is guided at one or both of its side edges.

While the invention has been illustrated and described in detail in the drawings and foregoing description, such illustration and description are to be considered illustrative or exemplary and not restrictive, the invention being not limited to the disclosed designs. Other embodiments and variants are understood, and can be achieved by those skilled in the art when carrying out the claimed invention, based on a study of the drawings, the disclosure and the appended claims. New embodiments or variants may be obtained by combining any of the above teachings.

For example, although the working heads of screwdrivers are described and depicted as hexa-lobe working heads, alternatively the working head may be shaped as a inbus (hexagonal) working head, Philips working head, etcetera, and correspond to a complementary shaped drive. A drive may be configured as an external drive, for example a first drive 14 protruding from the proximal end 23.

In the claims, the word "comprising" and "including" do not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

**LIST OF REFERENCE SIGNS**

| | | | |
|---|---|---|---|
| 1 | spinal bone fastener | 22 | Distal end |
| | assembly | 23 | Proximal end |
| 1' | spinal bone fastener | 30 | rod receiving head |
| | assembly | 30' | front loading head |
| 1" | spinal bone fastener | 30" | side loading head |
| | assembly | 30D | double head |
| 1‴ | spinal bone fastener assembly | 30O | rod receiving head for outer screw |
| 1ʺʺ | spinal bone fastener assembly | 31 | head top end |
| | | 32 | head bottom end |
| 1‴ʺ | spinal bone fastener assembly | 33 | rod receiving opening |
| | | 34 | front side |
| 2 | spinal rod | 35 | back side |
| 4 | setscrew | 36 | flexible elongate member retaining means |
| 5 | standard spinal screw | | |
| 7 | spine segments | 37 | rod setting or tightening mechanism |
| 7' | specific spine segment | | |
| 8 | spine | 38 | end seat |
| 8' | curved spine | 39a | extension |
| 8" | straightened spine | 39b | extension |
| 10 | bone fastener | 39c | extension |
| 10' | bone fastener | 40 | internal thread |
| 11 | bone fastener head portion | 40' | external thread |
| 12 | bone engaging shaft | 40a | internal thread |
| 13 | flexible elongate member | 40b | internal thread |
| | retaining means | 41 | central pocket |
| 14 | first drive | 42 | clamping seat |
| 15 | external thread | 43 | sidewall |
| 16 | central cannulation | 45 | seat |
| 17 | fenestration | 47 | anti-rotation element |
| 18 | internal thread | 48 | flat face |
| 20 | insertion depth stop | 49 | first drive shaped aperture |
| 21 | removal tool drive | 50 | stepped section (shoulder) |
| 51 | extension end | 86 | distal coupling portion |
| 52 | base | 87 | loop portion |
| 53 | neighboring rod receiving opening | 90 | Spacer |
| | | 100 | first rod receiving opening end |
| 54 | rod setting or tightening mechanism | | |
| | | 110 | bottom passage |
| 55 | internal or external breaking relief | 111 | side passage |
| | | 111a | first side passage |
| 60 | (outer) rod tightening means / screw thread | 111b | adjacent side passage |
| | | 120 | bone cement insertion cannula |
| 60' | encompassing outer screw | | |
| 60" | standard setscrew | 130 | intermediate inlay |
| 61 | main body | 130A | inlay for tether pedicle screw application |
| 62 | rod tightening means bottom side | | |
| | | 130B | inlay for growth related application |
| 63 | second drive | | |
| 64 | rod tightening mechanism / screw thread | 131 | second rod receiving opening |
| | | 132 | clamping surface |
| 65 | top side | 133 | screwdriver passage |
| 66 | inner thread | 134 | central locking element |
| 70 | (inner) rod tightening means | 135 | side slit |
| 71 | main body | 136 | upper surface |
| 72 | bottom side | 137 | bottom surface |
| 73 | third drive | 138a | proximally directed leg portion |
| 74 | outer thread | | |
| 75 | top side | 138b | proximally directed leg portion |
| 80 | flexible elongate member | | |
| 80dd | double sided tether band and double tensioning | 140 | insertion device |
| | | 140' | double sided tensioning insertion device |
| 80ds | double sided tether band and single sided tensioning | | |
| | | 141 | indicator |
| 81 | long elongated flexible body | 142 | tensioning knob |
| 82 | distance determining portion | 143 | worm |
| 83 | blocked tether portion | 144 | tether holding hook |
| 84 | bone fastener guided portion | 145 | internal wall |
| 85 | arrow = pulling direction | 146 | side wall |
| 147 | slit for spinal rod | 187 | enlarged stop portion |
| 148 | tensioning mechanism | 190 | transition area |
| 150 | force measuring device | 190a | transition area |
| 151 | turnable knob | 190b | transition area |
| 160 | first screwdriver | 260 | second screwdriver |
| 161 | screwdriver head | 261 | screwdriver head |
| 162 | screwdriver inbus/torx head | 262 | screwdriver inbus/torx head |
| 170 | assembly/disassembly key | 287 | enlarged stop portion |
| 171 | turnable knob | 300 | kit |
| 180 | tether band | 387 | enlarged stop portion |
| 181 | free strand | D1 | height difference TPS |
| 181a | free strand | D2 | height difference growth |
| 181b | free strand | OP | outer periphery |
| 182 | distance determining portion | X1 | first distance |
| 183 | blocked tether portion | X2 | second distance |
| 186 | eye | | |

## Claims

1. A spinal bone fastener assembly (1 to 1‴ʺʺ) to be engaged to a posterior spinal bone fastener system, the spinal bone fastener assembly (1) comprising
a bone fastener (10) having a bone fastener head portion (11), a bone engaging elongated shaft (12) and a first drive (14) for introducing the bone fastener (10) into a target bone,
a rod receiving head (30) configured to receive and at least partially encompass a spinal rod (2) in a first rod receiving opening with a central pocket having a clamping seat,
a tightening means (60) configured to exert a retaining and/or clamping force between the spinal rod (2) and the rod receiving head (30),
at least one flexible elongate member (80, 180) connecting or coupling the rod receiving head (30) with the bone fastener (10),
wherein,
- the bone fastener (10) further comprises at least one first flexible elongate member retaining means (13),
- the rod receiving head (30, 30', 30") comprises at least one second flexible elongate member retaining means (36) and a first rod setting or tightening mechanism portion (37),
- the tightening means (60, 70) comprises a second drive (63, 73) to engage the tightening means (60, 70) into the rod receiving head (30, 30', 30"),
- the spinal bone fastener assembly (1 to 1‴ʺʺ) has a second tightening mechanism portion (64) sized and shaped to engage with the first rod setting or tightening mechanism portion (37), and
- the flexible elongated member (80, 180) comprises a long elongated flexible body (81) which is sized and shaped to engage with first and second flexible elongate member retaining means (13, 36),
**characterized in that** at least a distance determining portion (82) of the long elongated flexible body (81) spans between the bone fastener head portion (11) and the rod receiving head bottom end (32), wherein the bone fastener head portion (11) is spaced at a adjusted distance (X1, X2) from the rod receiving head bottom end (32).

2. The spinal bone fastener assembly (1 to 1‴ʺʺ) according to claim 1, wherein a tightening means is configured as a main tightening means (60) sized and shaped to receive an inner tightening means (70), wherein optionally the inner and main tightening means (70, 60) are threadedly engaged by means of an inner thread (66) and an outer thread (75).

3. The spinal bone fastener assembly (1 to 1‴ʺʺ) according to claim 1 or claim 2, wherein an intermediate inlay (130) is arranged between the clamping seat (42) and at least one of the main or, if present, inner tightening means (60, 70).

4. The spinal bone fastener assembly (1 to 1‴ʺʺ) according to claim 3, wherein the intermediate inlay (130) comprises a second rod receiving opening (131) with a first depth (D1) which is smaller than the spinal rod diameter, or a second depth (D2) which is larger than the spinal rod diameter.

5. The spinal bone fastener assembly (1 to 1‴ʺʺ) according to any one claims 1 to 4, wherein the spinal bone fastener assembly comprises a locked configuration and an unlocked configuration,
wherein in the locked configuration the elongated flexible member (80, 180) is biased against the clamping seat (42) and translation of the flexible elongate member (80, 180) through at least one of the first and second flexible elongate member retaining means (13, 36) is inhibited.

6. The spinal bone fastener assembly (1 to 1‴ʺʺ) according to claim 2 and claim 3 and claim 5, wherein in said locked configuration the inner tightening means (70) is abutting with the spinal rod (2) and the flexible elongate member (80) is abutting with the intermediate inlay (130) and the clamping seat (42) or wherein in said locked configuration the inner tightening means (70) is abutting with the intermediate inlay (130) and the flexible elongate member (80) is abutting with the intermediate inlay (130) and the clamping seat (42).

7. The spinal bone fastener assembly (1 to 1‴ʺʺ) according to claim 2 and claim 5, wherein in said locked configuration the main tightening means (60) is abutting with the spinal rod (2) and the flexible elongate member (80, 180) is abutting with the spinal rod (2) and the clamping seat (42).

8. The spinal bone fastener assembly (1 to 1‴ʺʺ) according to claim 2 and claim 5, wherein in said locked configuration the spinal rod (2) is abutting with the first rod receiving opening end (100) and the main tightening means bottom side (62).

9. The spinal bone fastener assembly (1 to 1‴ʺʺ) according to any of the preceding claims, wherein the first and second tightening mechanism portion (37, 64) are configured as internal and external threads and form a tightening mechanism or coupling.

10. The spinal bone fastener assembly (1 to 1‴ʺʺ) according to any of the preceding claims, wherein at least one of the first or second flexible elongate member retaining means (13, 36) is oversized in comparison to the cross-section (C) of the flexible elongate member (80).

11. The spinal bone fastener assembly (1 to 1‴ʺʺ) according to any of the preceding claims, wherein at least one of the first or second flexible elongate member retaining means (13, 36) fully encompasses the flexible elongate member (80) to inhibit separation thereof.

12. The spinal bone fastener assembly (1 to 1‴ʺʺ) according to any of the preceding claims, wherein at least one of the first flexible elongate member retaining means (13) are directed top to bottom or are directed side to side.

13. The spinal bone fastener assembly (1 to 1‴ʺʺ) according to any of the preceding claims, wherein the rod receiving head (30, 30') comprises an end seat (38) which forms a depth insertion depth stop for the main rod tightening means (60).

14. The spinal bone fastener assembly (1 to 1‴ʺʺ) according to any of the preceding claims, wherein the outer periphery (OP) of the bone fastener head portion (11) is predominantly non-circular or non-rotational symmetric.

15. The spinal bone fastener assembly (1 to 1‴ʺʺ) according to any of the preceding claims, wherein the first drive (14) comprises an internal thread (18) for attachment of a bone cement insertion cannula (120).

16. The spinal bone fastener assembly (1 to 1‴ʺʺ) according to any of the preceding claims, wherein the flexible elongated member (80) is made of woven or braided strands of a biocompatible surgical suture material such as UHMWPE fibres, nylon fibres, Polyester fibres, PET fibres, or Stainless Steel, Cobalt Chromium or titanium(alloy) cables as well as polyaramide, carbon fibres or silk.

17. The spinal bone fastener assembly (1 to 1‴ʺʺ) according to any of the preceding claims, wherein the bone fastener (10) comprises an insertion depth stop (20) arranged adjacent to the distal side of the bone fastener head portion (11) and wherein said insertion depth stop (20) inhibits a too deep insertion.

18. The spinal bone fastener assembly (1 to 1‴ʺʺ) according to any of the preceding claims, wherein the rod receiving head (30, 30', 30") comprises rod tightening means (60) receiving extensions (39a, 39b) that project from the head top end (31).

19. The spinal bone fastener assembly (1 to 1‴ʺʺ) according to any of the preceding claims, wherein the rod receiving head (30) comprises a first drive shaped aperture (49) that extends through the head bottom end (32).

20. The spinal bone fastener assembly (1. 1') according to any of the preceding claims, wherein the flexible elongated member (80, 180) has a distal coupling end (86) for attachment to an insertion device (140).

21. The spinal bone fastener assembly (1 to 1‴ʺʺ) according to any of the preceding claims, wherein the flexible elongated member (80) is entering or exiting the rod receiving head from the head bottom end (32) and/or wherein the flexible elongated member (80) is entering or exiting the rod receiving head from the head outer wall (50).

22. The spinal bone fastener assembly (1 to 1‴ʺʺ) according to any of the preceding claims, wherein the spinal bone fastener assembly (1) comprises a spacer (90) arranged between the bone fastener (10) and the rod receiving head (30).

23. A kit comprising the spinal bone fastener assembly (1 to 1‴ʺʺ) of any of the preceding claims and an insertion device (140) including a tensioning mechanism (148) to tension the flexible elongate member (80, 180).

24. The kit of claim 23, furthermore comprising a tensioning force measuring device (150).

## Patentansprüche

1. Wirbelknochen-Fixierungsvorrichtung (1 bis 1‴ʺʺ), die dazu bestimmt ist, mit einem posterioren Wirbelknochen-Fixierungssystem in Eingriff zu kommen, wobei die Wirbelknochen-Fixierungsvorrichtung (1) umfasst:
eine Knochenbefestigung (10) mit einem Knochenbefestigungskopfabschnitt (11), einem länglichen Knocheneingriffsschaft (12) und einem ersten Antriebselement (14) zum Einbringen der Knochenbefestigung (10) in einen Zielknochen,
einen Stabaufnahmekopf (30), der so ausgebildet ist, dass er einen Wirbelsäulenstab (2) in einer ersten Stabaufnahmeöffnung aufnimmt und zumindest teilweise umschließt, wobei die Stabaufnahmeöffnung mit einer zentralen Tasche versehen ist, die einen Klemmsitz aufweist,
eine Klemmvorrichtung (60), die so ausgebildet ist, dass sie eine Halte- und/oder Klemmkraft zwischen dem Wirbelsäulenstab (2) und dem Stabaufnahmekopf (30) ausübt,
mindestens ein flexibles, längliches Element (80, 180), das den Stabaufnahmekopf (30) mit der Knochenbefestigung (10) verbindet oder koppelt,
wobei
- die Knochenbefestigung (10) ferner mindestens ein erstes Halteelement für ein flexibles, längliche Element (13) umfasst,
- der Stabaufnahmekopf (30, 30', 30") mindestens ein zweites Halteelement für das flexible, längliche Element (36) und einen ersten Stab-Positionierungs- oder Spannmechanismusabschnitt (37) umfasst,
- die Klemmvorrichtung (60, 70) ein zweites Antriebselement (63, 73) umfasst, um die Klemmvorrichtung (60, 70) in den Stabaufnahmekopf (30, 30', 30") einzurasten,
- die Wirbelknochen-Fixierungsvorrichtung (1 bis 1‴ʺʺ) weist einen zweiten Spannmechanismusabschnitt (64) auf, der so dimensioniert und geformt ist, dass er mit dem ersten Stab-Positionierungs- oder Spannmechanismusabschnitt (37) in Eingriff kommt, und
- das flexible, längliche Element (80, 180) einen flexiblen, länglichen Körper (81) umfasst, der so dimensioniert und geformt ist, dass er mit dem ersten und dem zweiten Halteelement für das flexible, längliche Element (13, 36) in Eingriff kommt,
**dadurch gekennzeichnet, dass** sich mindestens ein Abstandsbestimmungsabschnitt (82) des flexiblen, länglichen Körpers (81) zwischen dem Knochenbefestigungskopfabschnitt (11) und dem unteren Ende (32) des Stabaufnahmekopfes erstreckt, wobei der Knochenbefestigungskopfabschnitt (11) in einem eingestellten Abstand (X1, X2) zum unteren Ende (32) des Stabaufnahmekopfes angeordnet ist.

2. Wirbelknochen-Fixierungsvorrichtung (1 bis 1‴ʺʺ) nach Anspruch 1, wobei eine Klemmvorrichtung als Hauptklemmvorrichtung (60) ausgebildet ist, die so dimensioniert und profiliert ist, dass sie eine innere Klemmvorrichtung (70) aufnehmen kann, wobei die innere und die Hauptklemmvorrichtung (70, 60) gegebenenfalls über ein Innengewinde (66) und ein Außengewinde (75) in Gewindeeingriff stehen.

3. Wirbelknochen-Fixierungsvorrichtung (1 bis 1‴ʺʺ) nach Anspruch 1 oder Anspruch 2, wobei zwischen dem Klemmsitz (42) und mindestens einer der Haupt- oder, falls vorhanden, inneren Klemmvorrichtungen (60, 70) ein Zwischeneinsatz (130) angeordnet ist.

4. Wirbelknochen-Fixierungsvorrichtung (1 bis 1‴ʺʺ) gemäß Anspruch 3, wobei der Zwischeneinsatz (130) eine zweite Stabaufnahmeöffnung (131) umfasst, die eine erste Tiefe (D1) aufweist, die kleiner ist als der Durchmesser des Wirbelsäulenschafts, oder eine zweite Tiefe (D2), die größer ist als der Durchmesser des Wirbelsäulenschafts.

5. Wirbelknochen-Fixierungsvorrichtung (1 bis 1‴ʺʺ) gemäß einem der Ansprüche 1 bis 4, wobei die Wirbelknochen-Fixierungsvorrichtung eine verriegelte Konfiguration und eine entriegelte Konfiguration umfasst,
wobei das flexible, längliche Element (80, 180) in der verriegelten Konfiguration gegen den Klemmsitz (42) vorgespannt ist und die Verschiebung des flexiblen, länglichen Elements (80, 180) durch mindestens eines der ersten und zweiten Halteelemente für das flexible, längliche Element (13, 36) verhindert wird.

6. Wirbelknochen-Fixierungsvorrichtung (1 bis 1‴ʺʺ) gemäß Anspruch 2, Anspruch 3 und Anspruch 5, wobei die innere Klemmvorrichtung (70) in der genannten verriegelten Konfiguration an dem Wirbelsäulenstab (2) anliegt und das flexible, langgestreckte Element (80) an dem Zwischeneinsatz (130) und dem Klemmsitz (42) anliegt, oder die innere Klemmvorrichtung (70) in der genannten verriegelten Konfiguration an dem Zwischeneinsatz (130) anliegt und das flexible, langgestreckte Element (80) an dem Zwischeneinsatz (130) und dem Klemmsitz (42) anliegt.

7. Wirbelknochen-Fixierungsvorrichtung (1 bis 1‴ʺʺ) gemäß Anspruch 2 und Anspruch 5, wobei die Hauptklemmvorrichtung (60) in der genannten verriegelten Konfiguration an dem Wirbelsäulenstab (2) anliegt und das flexible, langgestreckte Element (80, 180) an dem Wirbelsäulenstab (2) und dem Klemmsitz (42) anliegt.

8. Wirbelknochen-Fixierungsvorrichtung (1 bis 1‴ʺʺ) gemäß Anspruch 2 und Anspruch 5, wobei der Wirbelsäulenstab (2) in der genannten verriegelten Konfiguration am Ende (100) der ersten Stabaufnahmeöffnung und an der Unterseite (62) der Hauptklemmvorrichtung anliegt.

9. Wirbelknochen-Fixierungsvorrichtung (1 bis 1‴ʺʺ) gemäß einem der vorhergehenden Ansprüche, wobei der erste und der zweite Spannmechanismusabschnitt (37, 64) als Innen- und Außengewinde ausgebildet sind und einen Spannmechanismus oder eine Kupplung bilden.

10. Wirbelknochen-Fixierungsvorrichtung (1 bis 1‴ʺʺ) gemäß einem der vorhergehenden Ansprüche, wobei mindestens eines der ersten oder zweiten Halteelemente für das flexible, längliche Element (13, 36) im Verhältnis zum Querschnitt (C) des flexiblen, länglichen Elements (80) überdimensioniert ist.

11. Wirbelknochen-Fixierungsvorrichtung (1 bis 1‴ʺʺ) gemäß einem der vorhergehenden Ansprüche, wobei mindestens eines der ersten oder zweiten Halteelemente für das flexible, längliche Element (13, 36) das flexible, längliche Element (80) vollständig umschließt, um dessen Ablösung zu verhindern.

12. Wirbelknochen-Fixierungsvorrichtung (1 bis 1‴ʺʺ) gemäß einem der vorhergehenden Ansprüche, wobei mindestens eines der ersten Halteelemente für ein flexibles, langgestrecktes Element (13) von oben nach unten oder von einer Seite zur anderen ausgerichtet ist.

13. Wirbelknochen-Fixierungsvorrichtung (1 bis 1‴ʺʺ) gemäß einem der vorstehenden Ansprüche, wobei der Stabaufnahmekopf (30, 30') einen Endsitz (38) umfasst, der einen Einführtiefenanschlag für die Hauptklemmvorrichtung (60) bildet.

14. Wirbelknochen-Fixierungsvorrichtung (1 bis 1‴ʺʺ) gemäß einem der vorhergehenden Ansprüche, wobei der äußere Umfang (OP) des Knochenbefestigungskopfabschnitts (11) im Wesentlichen nicht kreisförmig oder rotationsasymmetrisch ist ( ).

15. Wirbelknochen-Fixierungsvorrichtung (1 bis 1‴ʺʺ) gemäß einem der vorhergehenden Ansprüche, wobei das erste Antriebselement (14) ein Innengewinde (18) zur Befestigung einer Einführkanüle für Knochenzement (120) umfasst.

16. Wirbelknochen-Fixierungsvorrichtung (1 bis 1‴ʺʺ) gemäß einem der vorhergehenden Ansprüche, wobei das flexible, längliche Element (80) aus gewebten oder geflochtenen Strängen eines biokompatiblen chirurgischen Nahtmaterials besteht, wie beispielsweise UHMWPE-Fasern, Nylonfasern, Polyesterfasern, PET-Fasern oder Drähten aus Edelstahl, Kobalt-Chrom oder Titan (oder einer TitanLegierung) sowie aus Polyamid, Kohlenstofffasern oder Seide.

17. Wirbelknochen-Fixierungsvorrichtung (1 bis 1‴ʺʺ) gemäß einem der vorhergehenden Ansprüche, wobei die Knochenbefestigung (10) einen Einführtiefenanschlag (20) umfasst, der angrenzend an die distale Seite des Knochenbefestigungskopfabschnitts (11) angeordnet ist, und wobei der Einführtiefenanschlag (20) ein zu tiefes Einführen verhindert.

18. Wirbelknochen-Fixierungsvorrichtung (1 bis 1‴ʺʺ) gemäß einem der vorstehenden Ansprüche, wobei der Stabaufnahmekopf (30, 30', 30") Aufnahmeverlängerungen (39a, 39b) für eine Klemmvorrichtung (60) umfasst, die vom oberen Ende (31) des Kopfes vorstehen.

19. Wirbelknochen-Fixierungsvorrichtung (1 bis 1‴ʺʺ) gemäß einem der vorhergehenden Ansprüche, wobei der Stabaufnahmekopf (30) eine erste Öffnung (49) aufweist, die für ein Antriebselement profiliert ist, das sich durch das untere Ende (32) des Kopfes erstreckt.

20. Wirbelknochen-Fixierungsvorrichtung (1, 1') gemäß einem der vorhergehenden Ansprüche, wobei das flexible, längliche Element (80, 180) ein distales Kupplungsende (86) zur Befestigung an einer Einführvorrichtung (140) aufweist.

21. Wirbelknochen-Fixierungsvorrichtung (1 bis 1‴ʺʺ) gemäß einem der vorhergehenden Ansprüche, wobei das flexible, längliche Element (80) vom unteren Ende (32) des Stabaufnahmekopfes aus in den Stabaufnahmekopf eintritt oder aus diesem austritt und/oder das flexible, längliche Element (80) von der Außenwand (50) des Stabaufnahmekopfes aus in den Schaftaufnahmekopf eintritt oder aus diesem austritt.

22. Wirbelknochen-Fixierungsvorrichtung (1 bis 1‴ʺʺ) gemäß einem der vorhergehenden Ansprüche, wobei die Wirbelknochen-Fixierungsvorrichtung (1) einen Abstandhalter (90) umfasst, der zwischen der Knochenbefestigung (10) und dem Stabaufnahmekopf (30) angeordnet ist.

23. Bausatz, umfassend die Wirbelknochen-Fixierungsvorrichtung (1 bis 1‴ʺʺ) gemäß einem der vorhergehenden Ansprüche und eine Einführvorrichtung (140) mit einem Spannmechanismus (148) zum Spannen des flexiblen, länglichen Elements (80, 180).

24. Bausatz nach Anspruch 23, der ferner eine Vorrichtung zur Messung der Zugkraft (150) umfasst.

## Revendications

1. Ensemble de fixation d'os vertébral (1 à 1‴ʺʺ) destiné à venir en prise avec un système postérieur de fixation d'os vertébral, l'ensemble de fixation d'os vertébral (1) comprenant :
une fixation d'os (10) présentant une portion de tête de fixation d'os (11), une tige allongée de mise en prise avec l'os (12) et un premier organe d'entraînement (14) pour introduire la fixation d'os (10) dans un os cible,
une tête de réception de tige (30) configurée pour recevoir et englober au moins partiellement une tige vertébrale (2) dans une première ouverture de réception de tige pourvue d'une poche centrale présentant un siège de serrage,
un moyen de serrage (60) configuré pour exercer une force de retenue et/ou de serrage entre la tige vertébrale (2) et la tête de réception de tige (30),
au moins un élément allongé flexible (80, 180) reliant ou couplant la tête de réception de tige (30) à la fixation d'os (10),
dans lequel,
- la fixation d'os (10) comprend en outre au moins un premier moyen de retenue d'élément allongé flexible (13),
- la tête de réception de tige (30, 30', 30") comprend au moins un second moyen de retenue d'élément allongé flexible (36) et une première portion de mécanisme de mise en place ou de serrage de tige (37),
- le moyen de serrage (60, 70) comprend un second organe d'entraînement (63, 73) pour mettre en prise le moyen de serrage (60, 70) dans la tête de réception de tige (30, 30', 30"),
- l'ensemble de fixation d'os vertébral (1 à 1‴ʺʺ) présente une seconde portion de mécanisme de serrage (64) dimensionnée et profilée pour venir en prise avec la première portion de mécanisme de mise en place ou de serrage de tige (37), et
- l'élément allongé flexible (80, 180) comprend un corps flexible de grande longueur (81) qui est dimensionné et profilé pour venir en prise avec les premier et second moyens de retenue d'élément allongé flexible (13, 36),
**caractérisé en ce qu'**au moins une portion de détermination de distance (82) du corps flexible de grande longueur (81) s'étende entre la portion de tête de fixation d'os (11) et l'extrémité inférieure (32) de la tête de réception de tige, la portion de tête de fixation d'os (11) étant espacée d'une distance ajustée (X1, X2) par rapport à l'extrémité inférieure (32) de la tête de réception de tige.

2. Ensemble de fixation d'os vertébral (1 à 1‴ʺʺ) selon la revendication 1, un moyen de serrage étant configuré comme un moyen de serrage principal (60) dimensionné et profilé pour recevoir un moyen de serrage interne (70), les moyens de serrage interne et principal (70, 60) étant éventuellement en prise par filetage par l'intermédiaire d'un filetage interne (66) et d'un filetage externe (75).

3. Ensemble de fixation d'os vertébral (1 à 1‴ʺʺ) selon la revendication 1 ou la revendication 2, un insert intermédiaire (130) étant agencé entre le siège de serrage (42) et au moins un des moyens de serrage principal ou, s'il est présent, interne (60, 70).

4. Ensemble de fixation d'os vertébral (1 à 1‴ʺʺ) selon la revendication 3, l'insert intermédiaire (130) comprenant une seconde ouverture de réception de tige (131) présentant une première profondeur (D1) qui est inférieure au diamètre de la tige vertébrale, ou une seconde profondeur (D2) qui est supérieure au diamètre de la tige vertébrale.

5. Ensemble de fixation d'os vertébral (1 à 1‴ʺʺ) selon l'une quelconque des revendications 1 à 4, l'ensemble de fixation d'os vertébral comprenant une configuration verrouillée et une configuration déverrouillée,
l'élément allongé flexible (80, 180) étant, dans la configuration verrouillée, sollicité contre le siège de serrage (42), et la translation de l'élément allongé flexible (80, 180) à travers au moins un des premier et second moyens de retenue d'élément allongé flexible (13, 36) étant empêchée.

6. Ensemble de fixation d'os vertébral (1 à 1‴ʺʺ) selon la revendication 2, la revendication 3 et la revendication 5, le moyen de serrage interne (70) se mettant, dans ladite configuration verrouillée, en butée contre la tige vertébrale (2) et l'élément allongé flexible (80) se mettant en butée contre l'insert intermédiaire (130) et le siège de serrage (42), ou le moyen de serrage interne (70) se mettant, dans ladite configuration verrouillée, en butée contre l'insert intermédiaire (130) et l'élément allongé flexible (80) se mettant en butée contre l'insert intermédiaire (130) et le siège de serrage (42).

7. Ensemble de fixation d'os vertébral (1 à 1‴ʺʺ) selon la revendication 2 et la revendication 5, le moyen de serrage principal (60) se mettant, dans ladite configuration verrouillée, en butée contre la tige vertébrale (2) et l'élément allongé flexible (80, 180) se mettant en butée contre la tige vertébrale (2) et le siège de serrage (42).

8. Ensemble de fixation d'os vertébral (1 à 1‴ʺʺ) selon la revendication 2 et la revendication 5, la tige vertébrale (2) se mettant, dans ladite configuration verrouillée, en butée contre l'extrémité (100) de la première ouverture de réception de tige et le côté inférieur (62) du moyen de serrage principal.

9. Ensemble de fixation d'os vertébral (1 à 1‴ʺʺ) selon l'une quelconque des revendications précédentes, les première et seconde portions de mécanisme de serrage (37, 64) étant configurées comme des filetages internes et externes et formant un mécanisme de serrage ou un couplage.

10. Ensemble de fixation d'os vertébral (1 à 1‴ʺʺ) selon l'une quelconque des revendications précédentes, au moins un des premier ou second moyens de retenue d'élément allongé flexible (13, 36) étant surdimensionné par rapport à la section transversale (C) de l'élément allongé flexible (80).

11. Ensemble de fixation d'os vertébral (1 à 1‴ʺʺ) selon l'une quelconque des revendications précédentes, au moins un des premier ou second moyens de retenue d'élément allongé flexible (13, 36) englobant totalement l'élément allongé flexible (80) pour empêcher sa séparation.

12. Ensemble de fixation d'os vertébral (1 à 1‴ʺʺ) selon l'une quelconque des revendications précédentes, au moins un des premiers moyens de retenue d'élément allongé flexible (13) étant orienté de haut en bas ou orienté d'un côté à l'autre.

13. Ensemble de fixation d'os vertébral (1 à 1‴ʺʺ) selon l'une quelconque des revendications précédentes, la tête de réception de tige (30, 30') comprenant un siège d'extrémité (38) qui forme une butée de profondeur d'insertion pour le moyen principal de serrage de tige (60).

14. Ensemble de fixation d'os vertébral (1 à 1‴ʺʺ) selon l'une quelconque des revendications précédentes, la périphérie externe (OP) de la portion de tête de fixation d'os (11) étant majoritairement non circulaire ou asymétrique en rotation.

15. Ensemble de fixation d'os vertébral (1 à 1‴ʺʺ) selon l'une quelconque des revendications précédentes, le premier organe d'entraînement (14) comprenant un filetage interne (18) pour la fixation d'une canule d'insertion de ciment osseux (120).

16. Ensemble de fixation d'os vertébral (1 à 1‴ʺʺ) selon l'une quelconque des revendications précédentes, l'élément allongé flexible (80) étant constitué de brins tissés ou tressés d'un matériau de suture chirurgicale biocompatible tel que des fibres en UHMWPE, des fibres de nylon, des fibres de polyester, des fibres de PET, ou des câbles en acier inoxydable, en cobalt-chrome ou en titane (ou alliage), ainsi que de polyaramide, de fibres de carbone ou de soie.

17. Ensemble de fixation d'os vertébral (1 à 1‴ʺʺ) selon l'une quelconque des revendications précédentes, la fixation d'os (10) comprenant une butée de profondeur d'insertion (20) agencée de manière adjacente au côté distal de la portion de tête de fixation d'os (11), et ladite butée de profondeur d'insertion (20) empêchant une insertion trop profonde.

18. Ensemble de fixation d'os vertébral (1 à 1‴ʺʺ) selon l'une quelconque des revendications précédentes, la tête de réception de tige (30, 30', 30") comprenant des extensions de réception (39a, 39b) de moyen de serrage de tige (60) qui font saillie depuis l'extrémité supérieure (31) de la tête.

19. Ensemble de fixation d'os vertébral (1 à 1‴ʺʺ) selon l'une quelconque des revendications précédentes, la tête de réception de tige (30) comprenant une première ouverture (49) profilée pour un organe d'entraînement qui s'étend à travers l'extrémité inférieure (32) de la tête.

20. Ensemble de fixation d'os vertébral (1. 1') selon l'une quelconque des revendications précédentes, l'élément allongé flexible (80, 180) présentant une extrémité de couplage distale (86) pour une fixation à un dispositif d'insertion (140).

21. Ensemble de fixation d'os vertébral (1 à 1‴ʺʺ) selon l'une quelconque des revendications précédentes, l'élément allongé flexible (80) pénétrant dans la tête de réception de tige ou sortant de celle-ci depuis l'extrémité inférieure (32) de la tête et/ou l'élément allongé flexible (80) pénétrant dans la tête de réception de tige ou sortant de celle-ci depuis la paroi externe (50) de la tête.

22. Ensemble de fixation d'os vertébral (1 à 1‴ʺʺ) selon l'une quelconque des revendications précédentes, l'ensemble de fixation d'os vertébral (1) comprenant un espaceur (90) agencé entre la fixation d'os (10) et la tête de réception de tige (30).

23. Kit comprenant l'ensemble de fixation d'os vertébral (1 à 1‴ʺʺ) selon l'une quelconque des revendications précédentes et un dispositif d'insertion (140) incluant un mécanisme de mise en tension (148) pour mettre en tension l'élément allongé flexible (80, 180).

24. Kit selon la revendication 23, comprenant en outre un dispositif de mesure de force de tension (150).
